# EUROPEAN PATENT APPLICATION

(11) **EP 3 594 355 A1**
(43) Date of publication of application: **15.01.2020**
(21) Application number: 19184705.2
(22) Date of filing: 05.07.2019
(51) Int. Cl.: C12P 13/08, C07K 14/34, C12N 15/01

(54) **METHOD FOR THE FERMENTATIVE PRODUCTION OF L-LYSINE**

(30) Priority: 12.07.2018 EP 18183133; 05.09.2018 EP 18192624
(71) Applicant: Evonik Operations GmbH, 45128 Essen (DE)
(72) Inventor: Bekel, Thomas, 33790 Halle (Westf.) (DE); Schneider, Frank, 33790 Halle (DE); Thierbach, Georg, 33613 Bielefeld (DE); Voß, Kornelia, 33803 Steinhagen (DE)
(74) Representative: Evonik Patent Association

(57) **Abstract**

The present invention makes available a novel method for the fermentative production of L-lysine using bacteria of the species *Corynebacterium glutamicum,* having the ability to excrete L-lysine, containing in their chromosome a polynucleotide encoding a polypeptide having the activity of a transcriptional factor wherein an amino acid at a particular position of the amino acid sequence of the polypeptide has been substituted by a different proteinogenic amino acid.

## Description

L-lysine is used in human medicine, in the pharmaceutical industry, in the food industry and particularly in nutrition of animals. L-lysine is produced by fermentation of strains of the species *Corynebacterium glutamicum.* Because of the great economic importance, work is continually being done on improving the production methods. Improvements may relate to the fermentation technology such as e.g. stirring and supplying oxygen, or to the composition of the nutrient media e.g. the sugar concentration during fermentation, or to the processing of the fermentation broth to a suitable product form by e.g. drying and granulating the fermentation broth or ion exchange chromatography or may relate to the intrinsic performance properties of the microorganism itself.

The methods used for improving the performance properties of these microorganisms are those of mutagenesis, selection and screening of mutants. The strains obtained in this way are resistant to anti-metabolites or are auxotrophic for metabolites of regulatory importance, and produce L-lysine. A well-known anti-metabolite is the L-lysine analogue S-(2-aminoethyl)-L-cysteine (see e.g. Tosaka et al.: Agricultural and Biological Chemistry 42(4), 745-752, (1978)).
Methods of recombinant DNA technology have likewise been used for a number of years for improvement of L-lysine-producing strains of the species *Corynebacterium glutamicum,* by modifying, i.e. enhancing or attenuating, individual genes involved in L-lysine biosynthesis and investigating the effect on L-lysine production.

The nucleotide sequences of the chromosomes of various bacteria or strains resp. of the species *Corynebacterium glutamicum,* and their analysis have been disclosed. This information is available at publicly accessible databases and may be used for strain development purposes. One such database is the GenBank data base of the NCBI (National Center for Biotechnology Information, U.S. National Library of Medicine 8600 Rockville Pike, Bethesda MD, 20894 USA).

During the annotation procedure for a sequenced chromosome of an organism identified structures such as e.g. genes or coding sequences are furnished with a unique identifier called locus_tag by the supplier of the information to the data base.

The nucleotide sequence of the *Corynebacterium glutamicum* ATCC13032 chromosome and its analysis were described by Ikeda and Nakagawa (Applied Microbiology and Biotechnology 62, 99-109 (2003)) and in EP1108790. The information is available at the NCBI under accession number NC_003450. In the chromosome sequence disclosed under accession number NC_003450 locus_tag NCgl0458 identifies a nucleotide sequence coding for the transcription antitermination protein NusG. The amino acid sequence of the polypeptide is available under the identifier NP_599720.1. In EP1108790 the coding sequence is disclosed under sequence 532 (see GenBank accession number AX120616).

The nucleotide sequence of the *Corynebacterium glutamicum* ATCC13032 chromosome and its analysis were independently described by Kalinowski et al. (Journal of Biotechnology 104 (1-3), 5-25 (2003)). The information is available at the NCBI under accession number NC_006958. Locus_tag CGTRNA_RS02440-identifies a nucleotide sequence coding for the transcription termination/ antitermination protein NusG. The old_locus_tag designation cg0562 is also used in the art. The amino acid sequence of the polypeptide is available under the identifier WP_011013678. The amino acid sequence of the polypeptide is also available under accession number CAF19189, where it is described as transcription antitermination protein NusG.

The nucleotide sequences of locus_tag NCgl0458 and CGTRNA_RS02440 are identical.

Antitermination is a mechanism for controlling transcription by RNA polymerase in bacteria. During antitermination the activity of RNA polymerase is modified so that it reads beyond a transcriptional terminator into genes that lie downstream of said terminator.

The Encyclopedia of Genetics, Genomics, Proteomics and Informatics (Springer, Dordrecht, 2008; DOI: https://doi.org/10.1007/978-1-4020-6754-9_978) states: "Antitermination permits RNA polymerase to ignore transcription termination instructions such as bacterial rho and thus proceed through the termination signal."

A classical antiterminator known in bacterial genetics is the antiterminator protein N of *Escherichia coli* phage λ (lambda). Said antiterminator protein N permits the expression of phage genes allowing the virus to initiate its lytic phase. Protein N of phage λ forms a complex with host proteins called Nus (N (phage lambda protein) utilization substances) factors required to modify the activity of the RNA polymerase in such a way that it no longer responds to terminators. One of these Nus factors is the protein or polypeptide resp. NusG. The EcoCyc database (SRI International (Stanford Research Institute), Menlo Park, US, CA) for *Escherichia coli* summarizes under accession number EG10667 for the polypeptide encoded by the nusG gene: Transcription termination factor NusG is required for some kinds of Rho-dependent termination as well as for transcription antitermination.

Further readings concerning transcriptional termination and antitermination can be found in text books of microbiology, molecular biology or genetics, e.g. in the textbook Lewin's Genes XII by Jocelyn E. Krebs, Elliot S. Goldstein and Stephen T. Kilpatrick (Jones & Bartlett Learning, Burlington MA (US), 2018).

Barreiro et al. (Applied and Environmental Microbiology 67(5), 2183-2190, 2001) report on the organization and transcriptional analysis of a six-gene cluster of *Corynebacterium glutamicum* comprising the *nusG* gene. Barreiro et al. refer to the encoded polypeptide as antiterminator protein NusG.

The gene encoding the NusG polypeptide is called *nusG.* Information concerning transcription signals in *Corynebacterium glutamicum,* e.g. -10 region(s) of a promoter, or a transcriptional start site(s) of the *nusG* gene (old_locus_tag cg0562) can be found in Pfeifer-Sancar et al. (BMC Genomics 14:888 (2013)) or Barreiro et al..

The art describes the NusG polypeptide as a transcriptional antiterminator. Accordingly the NusG polypeptide has the activity of a polypeptide involved in transcription or the activity of a transcriptional factor resp..

Object of the present invention is to provide new measures for the fermentative production of L-lysine by bacteria of the species *Corynebacterium glutamicum.*

To achieve the object outlined above the present invention makes available a novel method for the fermentative production of L-lysine using bacteria of the species *Corynebacterium glutamicum,* having the ability to excrete L-lysine, containing in their chromosome a polynucleotide encoding a polypeptide having the activity of a transcriptional factor wherein the amino acid at position 210 of the amino acid sequence of the polypeptide contains any proteinogenic amino acid different from asparagine, preferably aspartic acid or glutamic acid instead of asparagine.

The present invention further makes available methods for the manufacturing of a product containing said L-lysine from the fermentation broth.

Accordingly, the present invention provides the following:
A method for the fermentative production of L-lysine comprising the steps of
a) providing a bacterium of the species *Corynebacterium glutamicum,* having the ability to excrete L-lysine, containing in its chromosome a polynucleotide encoding a polypeptide having the activity of a transcriptional factor and comprising the amino acid sequence of SEQ ID NO:2, wherein the amino acid asparagine at position 210 is substituted by a different proteinogenic amino acid, preferably by glutamic acid or aspartic acid, particularly preferred by aspartic acid,
b) cultivating the bacterium in a suitable medium under suitable conditions, and
c) accumulating the L-lysine in the medium to form an L-lysine containing fermentation broth.

It was found that the modified bacteria provided in the method according to the invention excreted L-lysine, into a suitable medium under suitable fermentation conditions in an increased manner with respect to one or more parameters selected from product concentration (i.e. amount of L-lysine produced per volume, or mass unit resp., of medium/fermentation broth (e.g. g/l or g/kg)), product yield (i.e. amount of L-lysine produced per carbon source consumed (e.g. g/g or kg/kg)), product formation rate (i.e. amount of L-lysine produced per volume, or mass unit resp., of medium/fermentation broth and unit of time (e.g. g/l x h or g/kg x h)), and specific product formation rate (i.e. amount of L-lysine produced per unit of time and mass unit of the producer (e.g. g/h x g dry mass)) as compared to the unmodified bacterium.

It is obvious that a higher product concentration facilitates product manufacturing e.g. purification and isolation. An increased product yield reduces the amount of raw material required. An increased product formation rate reduces the time required for a fermentation run thus increasing the availability of a given fermenter.

The method according to the invention thus contributes to the improvement of technical and economic aspects of the manufacturing of L-lysine or L-lysine containing products.

In one set of preferred embodiments the invention provides the following:
In a preferred embodiment the bacterium provided in the method according to the invention contains in its chromosome a polynucleotide encoding an amino acid sequence of a polypeptide having the activity of a transcriptional factor comprising the nucleotide sequence of positions 375 to 1328 of SEQ ID NO:1 the nucleobases from position 1002 to 1004 being gac or gat, preferably gac.

In another preferred embodiment the bacterium provided in the method according to the invention contains in its chromosome a polynucleotide encoding an amino acid sequence of a polypeptide having the activity of a transcriptional factor comprising the nucleotide sequence of positions 375 to 1331 of SEQ ID NO:1 the nucleobases from position 1002 to 1004 being gac or gat, preferably gac.

In another preferred embodiment the bacterium provided in the method according to the invention contains in its chromosome a polynucleotide encoding an amino acid sequence of a polypeptide having the activity of a transcriptional factor comprising the nucleotide sequence of positions 114 to 1331 of SEQ ID NO:1 the nucleobases from position 1002 to 1004 being gac or gat, preferably gac.

In another preferred embodiment the bacterium provided in the method according to the invention contains in its chromosome a polynucleotide encoding an amino acid sequence of a polypeptide having the activity of a transcriptional factor comprising the nucleotide sequence of positions 114 to 1402 of SEQ ID NO:1 the nucleobases from position 1002 to 1004 being gac or gat, preferably gac.

The nucleotide sequence shown in SEQ ID NO:3 is identical to that of SEQ ID NO:1 apart from the nucleobase at position 1002. The nucleobase at position 1002 of SEQ ID NO:1 is adenine. The nucleobase at position 1002 of SEQ ID NO:3 is guanine. Accordingly the nucleobases from positions 1002 to to 1004 of SEQ ID NO:3 are gac, a codon for aspartic acid. Accordingly the amino acid sequence of SEQ ID NO:4 contains the amino acid aspartic acid at position 210.

In another set of preferred embodiments the invention takes into account the degeneracy of the genetic code and provides the following:
In another preferred embodiment the bacterium provided in the method according to the invention contains in its chromosome a polynucleotide encoding an amino acid sequence of a polypeptide having the activity of a transcriptional factor comprising the nucleotide sequence of positions 375 to 1328 of SEQ ID NO:5.

In another preferred embodiment the bacterium provided in the method according to the invention contains in its chromosome a polynucleotide encoding an amino acid sequence of a polypeptide having the activity of a transcriptional factor comprising the nucleotide sequence of positions 375 to 1331 of SEQ ID NO:5.

In another preferred embodiment the bacterium provided in the method according to the invention contains in its chromosome a polynucleotide encoding an amino acid sequence of a polypeptide having the activity of a transcriptional factor comprising the nucleotide sequence of positions 114 to 1331 of SEQ ID NO:5.

In another preferred embodiment the bacterium provided in the method according to the invention contains in its chromosome a polynucleotide encoding an amino acid sequence of a polypeptide having the activity of a transcriptional factor comprising the nucleotide sequence of positions 114 to 1402 of SEQ ID NO:5.

The nucleotide sequence shown in SEQ ID NO:5 is identical to that of SEQ ID NO:3 with the following exceptions: The nucleobase at position 995 of SEQ ID NO:5 is cytosine resulting in a ttc codon for the amino acid phenylalanine at position 207 of the amino acid sequence. The nucleobase at position 998 of SEQ ID NO:5 is cytosine resulting in a gtc codon for the amino acid valine at position 208 of the amino acid sequence. The nucleobase at position 1001 of SEQ ID NO:5 is cytosine resulting in a ggc codon for the amino acid glycine at position 209 of the amino acid sequence. The nucleobase at position 1004 of SEQ ID NO:5 is thymine resulting in a gat codon for the amino acid aspartic acid at position 210 of the amino acid sequence.

Accordingly the amino acid sequence of SEQ ID NO:6 encoded by the nucleotide sequence of SEQ ID NO:5 positions 375 to 1328 is identical to that of SEQ ID NO:4 encoded by the nucleotide sequence of SEQ ID NO:3 positions 375 to 1328. The amino acid sequences of SEQ ID NO:4 and SEQ ID NO:6 contain the amino acid aspartic acid at position 210.

The term L-lysine, where mentioned herein, in particular in the context of product formation, also comprises their ionic forms and salts, for example L-lysine mono hydrochloride or L-lysine sulfate.

For practicing the present invention bacteria of the species *Corynebacterium glutamicum* are used. A description of the genus *Corynebacterium* and the species *Corynebacterium glutamicum* comprised by this genus can be found in the article *"*Corynebacterium" by K. A. Bernard and G. Funke in Bergey's Manual of Systematics of Archaea and Bacteria (Bergey's Manual Trust, 2012).

Suitable bacteria for the method of this invention are L-lysine excreting strains of *Corynebacterium glutamicum,* for example L-lysine excreting strains obtained by one or several steps of strain development from strain ATCC13032 and the like and modified as described in this invention.

Strain ATCC13032 (also available as DSM20300) is the taxonomic type strain of the species *Corynebacterium glutamicum.* A taxonomic study of this group of bacteria based on DNA-DNA hybridization was done by Liebl et al. (International Journal of Systematic Bacteriology 41(2), 255-260, 1991). A comparative analysis of various strains of the species *Corynebacterium glutamicum* based on genome sequence analysis was provided by Yang and Yang (BMC Genomics 18(1):940).

A multitude of L-lysine excreting strains of the genus *Corynebacterium,* in particular of the species *Corynebacterium glutamicum* were obtained in the art during the past decades starting from strains such as ATCC13032, ATCC14067, ATCC13869 and the like. They were obtained as a result of strain development programs using inter alia methods like classical mutagenesis, selection for antimetabolite resistance as well as amplification and promotor modification of genes of the biosynthetic pathway of the L-lysine by genetic engineering methods. Summaries may be found in L. Eggeling and M. Bott (Handbook of Corynebacterium glutamicum, CRC Press, 2005) or H. Yukawa and M. Inui (Corynebacterium glutamicum Biology and Biotechnology, Springer Verlag, 2013) or A. Yokota and M. Ikeda (Amino Acid Fermentation, Springer Verlag, 2017).

L-lysine excreting strains of the species *Corynebacterium glutamicum* are widely known in the art and can be modified as described in the present invention. For example Blombach et al. (Applied and Environmental Microbiology 75(2), 419-427, 2009) describe strain DM1933, which is deposited under accession DSM25442 according to the Budapest treaty. Strain DM1933 was obtained from ATCC13032 by several steps of strain development. Furthermore L-lysine excreting *Corynebacterium glutamicum* strain DM2031, deposited according to the Budapest Treaty as DSM32514 may be used. Strain DM2031 is a further developed derivative of DM1933 having enhanced L-lysine excretion ability. Other L-lysine excreting *Corynebacterium glutamicum* strains are e.g. described in WO2008033001 and EP0841395.

L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild strains like for example ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are for example given in US5688671, US6844176 and US6893848. A summarizing list can be found inter alia in WO2009141330. The symbol used in the art for a gene coding for an aspartokinase polypeptide is *lysC.* In case the gene codes for a feedback resistant polypeptide variant the art typically uses symbols like *lysC^{fbr}* with fbr indicating feedback resistance.

Accordingly said L-lysine excreting strains of the species *Corynebacterium glutamicum* modified as described in the present invention preferably contain at least (≥) one copy of a polynucleotide coding for a feedback resistant aspartokinase polypeptide.

SEQ ID NO:7 shows the nucleotide sequence of the coding sequence of the aspartokinase polypeptide of strain ATCC13032 and SEQ ID NO:8 the amino acid sequence of the encoded polypeptide. It is known in the art (see US6893848) that exchange of the amino acid Thr at position 311 of SEQ ID NO:8 for Ile imparts the enzyme feedback resistance to inhibition by mixtures of L-lysine and L-threonine.

Accordingly it is preferred that the amino acid sequence of said feedback resistant aspartokinase polypeptide comprises the amino acid sequence of SEQ ID NO:8 containing isoleucine at position 311.

Said amino exchange can be achieved by exchanging the nucleobase cytosine (c) at position 932 of SEQ ID NO:7 to give thymine (t). The acc codon for threonine is thus altered to the atc codon for isoleucine.

It is further known in the art that exchange of the gtg start codon of the coding sequence for the aspartokinase polypeptide for atg enhances expression of the polypeptide (see e.g. EP2796555).

Accordingly it is preferred that the sequence coding for a feedback resistant aspartokinase polypeptide begins with an atg start codon.

Summaries concerning the breeding of L-lysine excreting strains of *Corynebacterium glutamicum* may be found inter alia in L. Eggeling and M. Bott (Handbook of Corynebacterium glutamicum, CRC Press, 2005), V. F. Wendisch (Amino Acid Biosynthesis - Pathways, Regulation and Metabolic Engineering, Springer Verlag, 2007), H. Yukawa and M. Inui (Corynebacterium glutamicum Biology and Biotechnolgy, Springer Verlag, 2013), and Eggeling and Bott (Applied Microbiology and Biotechnology 99 (9), 3387-3394, 2015).

The term DSM denotes the depository Deutsche Sammlung für Mikroorganismen und Zellkulturen located in Braunschweig, Germany. The term ATCC denotes the depository American Type Culure Collection located in Manasass, Virginia, US.

Details regarding the biochemistry and chemical structure of polynucleotides and polypeptides as present in living things such as bacteria like *Corynebacterium glutamicum* or *Escherichia coli,* for example, can be found inter alia in the text book "Biochemie" by Berg et al. (Spektrum Akademischer Verlag Heidelberg, Berlin, Germany, 2003; ISBN 3-8274-1303-6).

Polynucleotides consisting of deoxyribonucleotide monomers containing the nucleobases or bases resp. adenine (a), guanine (g), cytosine (c) and thymine (t) are referred to as deoxyribopolynucleotides or deoxyribonucleic acid (DNA). Polynucleotides consisting of ribonucleotide monomers containing the nucleobases or bases resp. adenine (a), guanine (g), cytosine (c) and uracil (u) are referred to as ribo-polynucleotides or ribonucleic acid (RNA). The monomers in said polynucleotides are covalently linked to one another by a 3',5'-phosphodiester bond. By convention single strand polynucleotides are written from 5'- to 3'-direction. Accordingly a polynucleotide has a 5'-end and 3'-end. The order of the nucleotide monomers in the polynucleotide is commonly referred to as nucleotide sequence. Accordingly a polynucleotide is characterized by its nucleotide sequence. For the purpose of this invention deoxyribopolynucleotides are preferred. In bacteria, for example *Corynebacterium glutamicum* or *Escherichia coli,* the DNA is typically present in double stranded form. Accordingly the length of a DNA molecule is typically given in base pairs (bp). The nucleotide sequence coding for a specific polypeptide is called coding sequence (cds). A triplett of nucleotides specifying an amino acid or a stop signal for translation is called a codon. The codons specifying different amino acids are well known in the art.

A gene from a chemical point of view is a polynucleotide, preferably a deoxyribopolynucleotide.

DNA can be synthesized *de novo* by the phoshoramidite method (McBride and Caruthers: Tetrahedon Letters 24(3) 245- 248 (1983)). Summaries concerning *de novo* DNA synthesis may be found in the review article of Kosuri and Church (Nature Methods 11(5), 499 - 507 (2014)) or in the article of Graf et al. in the book "Systems Biology and Synthetic Biology" by P. Fu and S. Panke (John Wiley, US, 2009, pp 411-438).

The term gene refers to a polynucleotide comprising a nucleotide sequence coding for a specific polypeptide (coding sequence) and the adjoining stop codon. In a broader sense, the term includes regulatory sequences preceding and following the coding sequence. The preceding sequence is located at the 5'-end of the coding sequence and is also referred to as upstream sequence. A promotor is an example of a regulatory sequence located 5' to the coding sequence. The sequence following the coding sequence is located at its 3'-end and also referred to as downstream sequence. A transcriptional terminator is an example of a regulatory sequence located 3' to the coding sequence.

Polypeptides consist of L-amino acid monomers joined by peptide bonds. For abbreviation of L-amino acids the one letter code and three letter code of IUPAC (International Union of Pure and Applied Chemistry) is used. Due to the nature of polypeptide biosynthesis polypeptides have an amino terminal end and a carboxyl terminal end also referred to as N-terminal end and C-terminal end. The order of the L-amino acids or L-amino acid residues resp. in the polypeptide is commonly referred to as amino acid sequence. Polypeptides are also referred to as proteins.
Further it is known in the art that the start codon or initiation codon resp. gtg of a coding sequence as well as atg encodes the amino acid methionine. It is known in the art that the N-terminal amino acid methionine of an encoded polypeptide may be removed by an aminopeptidase during or after translation (Jocelyn E. Krebs, Elliott S. Goldstein and Stephan T. Kilpatrick: Lewin's Genes X, Jones and Bartlett Publishers, US, 2011). WO0202778 describes the isolation, purification and N-terminal sequencing of the malate dehydrogenase of *Corynebacterium glutamicum* ATCC13032.

Proteinogenic L-amino acids are to be understood to mean the L-amino acids present in natural proteins, that are proteins of microorganisms, plants, animals and humans. Proteinogenic L-amino acids comprise L-aspartic acid, L-asparagine, L-threonine, L-serine, L-glutamic acid, L-glutamine, L-glycine, L-alanine, L-cysteine, L-valine, L-methionine, L-isoleucine, L-leucine, L-tyrosine, L-phenylalanine, L-histidine, L-lysine, L-tryptophan, L-arginine, L-proline and in some cases L-selenocysteine and L-pyrrolysine. It is common in the ars to refer to these proteinogenic L-amino acids without hinting to the L configuration at the α carbon atom of the L-amino acid; e.g. L-asparagine may simply called asparagine, L-aspartic acid may simply be called aspartic acid etc..

Teachings and information concerning the handling of and experimental work with polynucleotides may be found inter alia in the handbook of J. Sambrook et al. (Molecular Cloning, A Laboratory Manual, Cold Spring Harbor Laboratory Press, 1989), the textbook of C. R. Newton and A. Graham (PCR, Spektrum Akademischer Verlag, 1994) and the handbook of D. Rickwood and B. D. Hames (Gel electrophoresis of nucleic acids, a practical approach, IRL Press, 1982).

For sequence analysis of polynucleotides and polypeptides, e.g. sequence alignments the Clustal W program (Larkin et al.: Clustal W and Clustal X version 2.0. In: Bioinformatics 23, 2947-2948 (2007)) or public software such as the CLC Genomics Workbench (Qiagen, Hilden, Germany) or the program MUSCLE provided by the European Bioinformatics Institute (EMBL-EBI, Hinxton, UK) may be used.

*Corynebacterium glutamicum,* in particular strain ATCC13032 and L-lysine excreting strains obtained therefrom during a strain development program, contain in their chromosome a, in particular one, gene encoding a polypeptide having the activity of a transcriptional factor, also referred to as transcriptional antiterminator in the art, and comprising the amino acid sequence of SEQ ID NO:2. The coding sequence is shown in SEQ ID NO:1 positions 375 to 1328. The coding sequence may contain silent mutations which do not alter the amino acid sequence of the NusG polypeptide. This context is also known as degeneracy of the genetic code in the art. The promoter and a stem-and-loop structure indicating a terminator described in the art are depicted in the sequence listing.

During the work for the present invention it was found that modifying L-lysine excreting bacteria of the species *Corynebacterium glutamicum* by exchanging the amino acid asparagine at position 210 of the encoded amino acid sequence of the NusG polypeptide shown in SEQ ID NO:2 for a different proteinogenic amino acid, preferably aspartic acid or glutamic acid, particular preferred aspartic acid, increased their ability to excrete L-lysine in a fermentative process as compared to the unmodified bacterium.

The skilled partisan is aware of a number of methods of mutagenesis how to achieve said modification in the *Corynebacterium glutamicum.*

A mutant bacterium according to the invention can be obtained by classical *in vivo* mutagenesis executed with cell populations of strains of *Corynebacterium glutamicum* using mutagenic substances, e.g. N-methyl-N'-nitro-N-nitrosoguanidine, or ultra violet light.

The nucleotide sequence comprising the site of mutagenesis within the *nusG* gene can be amplified by PCR using primers selected from SEQ ID NO:1 or SEQ ID NO:3. By sequencing the PCR product the desired mutants are identified. Details concerning this approach can be found inter alia in US7754446. Real-time PCR in combination with FRET hybridization probes may also be used for mutation detection. The term FRET is the abbreviation for fluorescence resonance energy transfer. Cyril D S Mamotte (The Clinical Biochemist Reviews 27, 63-75 (2006)) reviews the identification of single nucleotide substitutions using this method. Further summaries concerning this method may be found in the textbook Lewin's Genes XII by Jocelyn E. Krebs, Elliott S. Goldstein and Stephan T. Kilpatrick (Jones and Bartlett Publishers, US, 2018) or elsewhere in the art.

A further method of mutagenesis is the CRISPR-Cpf1 assisted genome editing described by Jiang et al. (Nature Communications |8:15179| DOI:10.1038/ncomms15179) or the CRISPR-Cas9 assisted genome editing described by Cho et al. (Metabolic Engineering, 2017 Jul;42:157-167. doi: 10.1016/j.ymben.2017.06.010.), Peng etal. (Microbial Cell Factories, 2017 Nov 14;16(1):201. doi: 10.1186/s12934-017-0814-6.) and Liu et al. (Microbial Cell Factories, 2017 Nov 16; 16(1):205. doi: 10.1186/s12934-017-0815-5.)

Another common method of mutating genes of *Corynebacterium glutamicum* is the method of gene replacement described by Schwarzer and Pühler (Bio/Technology 9, 84-87 (1991)) and further elaborated by Schafer et al. (Gene 145, 69-73 (1994)).

Peters-Wendisch et al. (Microbiology 144, 915-927 (1998)) used the gene replacement method to inactivate the *pyc* gene of *Corynebacterium glutamicum* encoding pyruvate carboxylase. In US7585650 the method was applied to the *zwf* gene to realize an amino acid exchange at position 321 of the amino acid sequence of the Zwf sub-unit of the glucose 6-phosphate dehydrogenase. In US7754446 the method was applied to the *rel* gene to realize an amino acid exchange at position 38 of the amino acid sequence of the GTP- pyrophosphate kinase polypeptide.

In the gene replacement method, a mutation, for example, a deletion, insertion or substitution of at least one nucleobase, is provided by an isolated polynucleotide comprising the nucleotide sequence of the gene in question or a part thereof containing the mutation.

In the context of the present invention the nucleotide sequence of the gene in question is the *nusG* gene.

In the context of the present invention the mutation is a substitution of at least one nucleobase located in the codon specifying the amino acid asparagine at position 210 of the encoded amino acid sequence (see SEQ ID NO:1 and SEQ ID NO:2) of the NusG polypeptide.

As a consequence of said mutation the codon specifies a proteinogenic amino acid different from asparagine, preferably aspartic acid or glutamic acid, particular preferred aspartic acid. The codons specifying aspartic acid are gac or gat. The codon gac is preferred.

The codon for the amino acid at position 210 has the position from 1002 to 1004 in SEQ ID NO:1 or SEQ ID NO:3. The nucleotide sequence from position 1002 to 1004, in particular the nucleotide at position 1002, may also be referred to as site of mutation.

The mutated nucleotide sequence of the gene in question or a part thereof containing the mutation comprises i) a nucleotide sequence at the 5'-end of the site of mutation, which is also referred to as 5'-flanking sequence or upstream sequence in the art, ii) a nucleotide sequence at the 3'-end of the site of mutation, which is also referred to as 3'-flanking sequence or downstream sequence in the art, and iii) the nucleotide sequence of the site of mutation between i) and ii).

Said 5'-flanking sequence and 3'-flanking sequence required for homologous recombination typically have a length of at least 200 bp, at least 400 bp, at least 600 bp or at least 800 bp. The maximum length typically is 1000 bp, 1500 bp or 2000 bp.

An example of a mutated nucleotide sequence in the context of the present invention comprises the nucleotide sequence shown in SEQ ID NO:1 from positions 114 to 1402 with guanine at position 1002 or comprises the nucleotide sequence shown in SEQ ID NO:3 from positions 114 to 1402.

Another example of a mutated nucleotide sequence in the context of the present invention comprises the nucleotide sequence shown in SEQ ID NO:1 from positions 205 to 1802 with guanine at position 1002, or comprises the nucleotide sequence shown in SEQ ID NO:3 from positions 205 to 1802 resp..

The nucleotide sequence of SEQ ID NO:9 from positions 8 to 1605 is identical to the nucleotide sequence of SEQ ID NO:3 from positions 205 to 1802. SEQ ID NO: 9 contains a recognition site for the restriction endonuclease *XbaI* at the 5'-end and at the 3'-end useful for cloning purposes.

Said 5'-flanking sequence consists of the nucleotide sequence from positions 8 to 804 of SEQ ID NO:9. The 3'-flanking sequence consists of the nucleotide sequence from positions 806 to 1605 of SEQ ID NO:9. The site of mutation is at position 805 of SEQ ID NO:9.

The mutated nucleotide sequence provided is cloned into a plasmid vector, e.g. pK18mobsacB described by Schafer et al. (Gene 145, 69-73 (1994)), that is not capable of autonomous replication in *Corynebacterium glutamicum.* Said plasmid vector comprising said mutated nucleotide sequence is subsequently transferred into the desired strain of *Corynebacterium glutamicum* by transformation using electroporation or conjugation. After two events of homologous recombination comprising a recombination event within the 5'-flanking sequence provided by the plasmid vector with the homologous sequence of the *Corynebacterium glutamicum* chromosome and a recombination event within the 3'-flanking sequence provided by the plasmid vector with the homologous sequence of the *Corynebacterium glutamicum* chromosome, one effecting integration and one effecting excision of said plasmid vector, the mutation is incorporated in the *Corynebacterium glutamicum* chromosome. Thus the nucleotide sequence of the gene in question contained in the chromosome of said desired strain is replaced by the mutated nucleotide sequence. The presence of the mutation in the desired strain is then confirmed e.g. by analysis of the nucleotide sequence or real-time PCR using FRET as described above.

An event of homologous recombination may also be referred to as crossing over.

It is preferred that the L-lysine excreting *Corynebacterium glutamicum* strains provided for the method of the present invention have the ability to excrete ≥ 0,25 g/l, preferably ≥ 0,5 g/l, particularly preferred ≥ 1,0 g/l, very particularly preferred ≥ 2,0 g/l of L-lysine in a suitable medium under suitable conditions.

In a fermentative process according to the invention a *Corynebacterium glutamicum* modified in accordance with the present invention and having the ability to excrete L-lysine is cultivated in a suitable medium under suitable conditions. Due to said ability to excrete said L-lysine the concentration of the L-lysine increases and accumulates in the medium during the fermentative process and the L-lysine is thus produced.

The fermentative process may be a discontinuous process like a batch process or a fed batch process or a continuous process. A summary concerning the general nature of fermentation processes is available in the textbook by H. Chmiel (Bioprozesstechnik, Spektrum Akademischer Verlag, 2011), in the textbook of C. Ratledge and B. Kristiansen (Basic Biotechnology, Cambridge University Press,2006) or in the textbook of V.C. Hass and R. Pörtner (Praxis der Bioprozesstechnik Spektrum Akademischer Verlag, 2011).

A suitable medium used for the production of L-lysine by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasses or high fructose corn syrup.

As nitrogen source organic nitrogen-containing compounds such as peptones, meat extract, soy bean hydrolysates or urea, or inorganic compounds such as ammonium sulphate, ammonium chloride, ammonium phosphate, ammonium carbonate, ammonium nitrate, ammonium gas or aqueous ammonia can be used.
As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds means essential growth factors like vitamins e. g. thiamine or biotin or L-amino acids e.g. L-homoserine.

The media components may be added to the culture in form of a single batch or be fed in during the cultivation in a suitable manner.

During the fermentative process, the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the L-lysine sufficient for being recovered has been formed. The cultivation is then completed. This aim is normally achieved within 10 hours to 160 hours. In continuous processes, longer cultivation times are possible.

Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

Thus the fermentative process results in a fermentation broth which contains the desired L-lysine.

A product containing the L-lysine is then recovered or manufactured in liquid or solid from the fermentation broth.

A "fermentation broth" means a medium in which a *Corynebacterium glutamicum* described in the invention has been cultivated for a certain time and under certain conditions.

When the fermentative process is completed, the resulting fermentation broth accordingly comprises:
a) the biomass (cell mass) of the *Corynebacterium glutamicum* of the invention, said biomass having been produced due to propagation of the cells of said *Corynebacterium glutamicum,*
b) the desired L-lysine accumulated during the fermentative process,
c) the organic by-products accumulated during the fermentative process, and
d) the components of the medium employed which have not been consumed in the fermentative process.

The organic by-products include compounds which may be formed by the *Corynebacterium glutamicum* of the invention during the fermentative process in addition to production of the L-lysine.

The fermentation broth is removed from the culture vessel or fermentation tank, collected where appropriate, and used for providing a product containing the L-lysine, in liquid or solid form. The expression "recovering the L-lysine-containing product" is also used for this. In the simplest case, the L-lysine -containing fermentation broth itself, which has been removed from the fermentation tank, constitutes the recovered product.

The fermentation broth can subsequently be subjected to one or more of the measures selected from the group consisting of:
a) partial (>0% to <80%) to complete (100%) or virtually complete (≥ 80%, ≥ 90%, ≥ 95%, ≥ 96%, ≥ 97%, ≥ 98%, ≥ 99%) removal of the water,
b) partial (>0% to <80%) to complete (100%) or virtually complete (≥ 80%, ≥ 90%, ≥ 95%, ≥ 96%, ≥ 97%, ≥ 98%, ≥ 99%) removal of the biomass, the latter being optionally inactivated before removal,
c) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the organic by-products formed during the fermentative process, and
d) partial (>0% to <80%) to complete (100%) or virtually complete (≥ 80%, ≥ 90%, ≥ 95%, ≥ 96%, ≥ 97%, ≥ 98%, ≥ 99%, ≥ 99.3%, ≥ 99.7%) removal of the residual components of the medium employed or of the residual input materials resp., which have not been consumed in the fermentative process.

An abundance of technical instructions for measures a), b), c) and d) are available in the art.

Removal of water (measure a)) can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Accordingly a method according to the invention comprises extracting or substantially eliminating water from said fermentation broth. In particular at least 40 % (w/w), preferred at least 90 % (w/w), more preferred at least 95 % (w/w) water are extracted from the fermentation broth.

Removal of the biomass (measure b)) can be achieved inter alia by centrifugation, filtration or decantation or a combination thereof.

Removal of the organic by-products (measure c)) or removal of residual components of the medium (measure d) can be achieved inter alia by chromatography, e.g. ion exchange chromatography, treatment with activated carbon or crystallization. In case the organic by-products or residual components of the medium are present in the fermentation broth as solids they can be removed by measure b).

Accordingly the manufacturing of an L-lysine product according to the invention comprises a purification step, preferably selected from the group consisting ion exchange chromatography, treatment with activated carbon or crystallization.

Thus e. g. a product containing L-lysine x HCl, preferably containing ≥ 80 % L-lysine x HCl, particularly preferred ≥ 90 % L-lysine x HCl or ≥ 95 % L-lysine x HCl can be obtained.

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US5431933 teaches the manufacturing of dry L-amino acid products, e. g. an L-lysine product, containing most of the constituents of the fermentation broth.

Thus a concentration or purification of the L-lysine is achieved and a product having the desired content of said L-lysine is provided.

Analysis of L-lysine to determine its concentration at one or more time(s) during the fermentation can take place by separating the L-lysine by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

### EXPERIMENTAL SECTION

### A) MATERIALS and METHODS

The molecular biology kits, primers and chemicals used and some details of the methods applied are briefly described herewith.

### 1. Antibiotics and chemicals

a. Kanamycin: Kanamycin solution from *Streptomyces kanamyceticus* from Sigma Aldrich (St. Louis, USA, Cat. no. K0254).
b. Nalidixic acid: Nalidixic acid sodium salt from Sigma Aldrich (St. Louis, USA, Cat. no. N4382).
c. If not stated otherwise, all chemicals were purchased analytically pure from Merck (Darmstadt, Germany), Sigma Aldrich (St. Louis, USA) or Carl-Roth (Karlsruhe, Germany).

### 2. Cultivation

If not stated otherwise, all cultivation / incubation procedures were performed as follows herewith:
a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 37°C and 200 rpm.
b. LB agar (MILLER) from Merck (Darmstadt, Germany Cat. no. 110283) was used for cultivation of *E. coli* strains on agar plates. The agar plates were incubated at 37°C in an INCU-Line® mini incubator from VWR (Radnor, USA).
c. Brain heart infusion broth (BHI) from Merck (Darmstadt, Germany; Cat. no. 110493) was used to cultivate *C*. *glutamicum* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 33°C and 200 rpm.
d. Brain heart agar (BHI-agar) from Merck (Darmstadt, Germany; Cat. no. 113825) was used for cultivation of *C*. *glutamicum* strains on agar plates. The agar plates were incubated at 33°C in an incubator from Heraeus Instruments with Kelvitron® temperature controller (Hanau, Germany).

### 3. Determining optical density

a. The optical density of bacterial suspensions in shake flask cultures was determined at 600 nm (OD600) using the BioPhotometer from Eppendorf AG (Hamburg, Germany).
b. The optical density of bacterial suspensions produced in the Wouter Duetz (WDS) micro fermentation system (24-Well Plates) was determined at 660 nm (OD660) with the GENios™ plate reader from Tecan Group AG (Männedorf, Switzerland).

### 4. Centrifugation

a. Benchtop centrifuge for reaction tubes with a volume up to 2 ml
   Bacterial suspensions with a maximum volume of 2 ml were caused to sediment using 1 ml or 2 ml reaction tubes (e.g. Eppendorf Tubes® 3810X) using an Eppendorf 5417 R centrifuge (5 min. at 13.000 rpm).
b. Benchtop centrifuge for tubes with a volume up to 50 ml
   Bacterial suspensions with a maximum volume of 50 ml were caused to sediment using 15 ml or 50 ml centrifuge tubes (e.g. Falcon™ 50 ml Conical Centrifuge Tubes) using an Eppendorf 5810 R centrifuge for 10 min. at 4.000 rpm.

### 5. Detection of mutations using FRET

The presence of a given mutation, e.g. a nucleobase exchange, was detected by real-time PCR in combination with FRET hybridization probes. The term FRET is the abbreviation for fluorescence resonance energy transfer. As real-time PCR instrument a Lightcycler from Roche Diagnostics® was used (see below).

This method was e. g. used by M. J. Lay and C. T. Wittwer (Clinical Chemistry 42 (12), 2262-2267 (1997)) for the genotyping of factor V Leiden. Cyril DS Mamotte (The Clinical Biochemist Reviews 27, 63-75 (2006) reviews the genotyping of single nucleotide substitutions using this method.

Summaries concerning this method may be found in the textbooks Lewin's Genes XII by Jocelyn E. Krebs, Elliott S. Goldstein and Stephan T. Kilpatrick (Jones and Bartlett Publishers, US, 2018), Molecular Diagnostics, 12 Tests that changed everything by W. Edward Highsmith (Humana Press, Springer, New York, 2014) or elsewhere in the art.

The FRET hybridization donor probe was labelled with the fluorescent dye fluorescein and the acceptor probe with the fluorescent dye LC-Red640. In essence the detection method comprised three steps: colony PCR, probe hybridization and subsequent melting curve analysis. The method is simply referred to as real-time PCR herewith.

### a. Primers and Probes

The oligonucleotides used were synthesized by eurofins genomics GmbH (Ebersberg, Germany).

### b. Template

As PCR template the total DNA contained in a colony was used. It was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.

### b. Reaction Mix

The Type-it® Fast SNP probe PCR Kit (Type-it Kit) from Qiagen (Hilden, Germany, Cat.No. 206045) was used for real-time detection of the mutations. Therefore 2.5 µl of the Qiagen Fast SNP Puffer (2x) was mixed with 0.5 µl of each of the LC-PCR-Primers [10 µM] and 0.5 µl of each of the 1:500 diluted acceptor and donor probe [100 pmol/µl] to get the mastermix for the real-time PCR.

**Table 1: Thermocycling conditions for PCR with the LightCycler® (step 1-3) and melting curve analysis (step 4-6).**

| PCR-program | | | |
|---|---|---|---|
| Step | Time [sec.] | T [°C] | Description |
| 1 | 15 | 95 | Denaturation step (and Activation of HotStarTaq™ DNA polymerase) |
| 2 | 05 | 55 | Annealing step |
| 3 | 30 | 72 | Elongation step |
| | | | Repeat step 1 to 3: 50 x |
| 4 | 10 | 95 | Denaturation step |
| 5 | 30 | 40 | Probe hybridisation |
| 6 | | 40- 80 | Melting curve analysis |
| 7 | | 80- 40 | Cooling |

### c. PCR Cycler

The reactions were carried out in a LightCycler® 2.0 Instrument and analysed with LightCycler® Software 4.1 of Roche Diagnostics (Rotkreuz, Switzerland).

### 6. Chemical transformation of E. coli

*E. coli* K-12 strain S17-1 was used as donor for conjugational transfer of plasmids based on pK18mobsacB from *E. coli* to *C*. *glutamicum.* Strain S17-1 is described by Simon, R. et al. (Bio/Technology 1, 784-794, 1983). It is available from the American Type Culture Collection under the accession number ATCC47055.

Chemically competent *E. coli* S17-1 cells were made as follows: A preculture of 10 ml LB medium (10 ml liquid medium per 100 ml Erlenmeyer flask with 3 baffles) was inoculated with 100 µl bacterial suspension of strain S17-1 and the culture was incubated overnight for about 18 h at 37°C and 250 rpm. The main culture (70 ml LB contained in a 250 ml Erlenmeyer flask with 3 baffles) was inoculated with 300 µl of the preculture and incubated up to an OD600 of 0.5-0.8 at 37°C. The culture was centrifuged for 6 min. at 4°C and 4000 rpm and the supernatant was discarded. The cell pellet was resuspended in 20 ml sterile, ice-cold 50 mM CaCl₂ solution and incubated on ice for 30 min.. After another centrifugation step, the pellet was resuspended in 5 ml ice-cold 50 mM CaCl₂ solution and the suspension incubated on ice for 30 min.. The cell suspension was then adjusted to a final concentration of 20 % glycerol (v/v) with 85 % (v/v) sterile ice-cold glycerol. The suspension was divided into 50 µl aliquots and stored at -80°C.

To transform S17-1 cells, the protocol according to Tang et al. (Nucleic Acids Res. 22(14), 2857-2858, 1994) with a heat shock of 45 sec. was used.

### 7. Conjugation of C. glutamicum

The pK18mobsacB plasmid system described by Schafer et al. (Gene 145, 69 - 73, 1994) was used to integrate desired DNA fragments into the chromosome of *C. glutamicum.* A modified conjugation method of Schafer et al. (Journal of Bacteriology 172, 1663 - 1666, 1990) was used to transfer the respective plasmid into the desired *C. glutamicum* recipient strain.

Liquid cultures of the *C. glutamicum* strains were carried out in BHI medium at 33°C. The heat shock was carried out at 48.5°C for 9 min.. Transconjugants were selected by plating the conjugation batch on EM8 agar (Table 2), which was supplemented with 25 mg/l kanamycin and 50 mg/l nalidixic acid. The EM8 agar plates were incubated for 72 h at 33°C.

**Table 2: Composition of the EM8 agar**

| **Components** | **Concentration (g/l)** |
|---|---|
| Glucose (sterile-filtered) | 23 |
| CSL (corn steep liquor; Roquette; solid content 48±2 % w/w) | 30 |
| Peptone from soymeal (Merck, Germany) | 40 |
| (NH₄)₂SO₄ | 8 |
| Urea | 3 |
| KH₂PO₄ | 4 |
| MgSO₄ · 7 H₂O | 0.5 |
| FeSO₄ · 7 H₂O | 0.01 |
| CuSO₄ · 5 H₂O | 0.001 |
| ZnSO₄ · 7 H₂O | 0.01 |
| Calcium pantothenate, D(+) | 0.01 |
| Thiamine | 0.001 |
| Inositol | 0.1 |
| Nicotinic acid | 0.001 |
| Biotin (sterile-filtered) | 0.005 |
| CaCO₃ (autoclaved separately) | 1.6 |
| Agar-Agar (Merck, Germany) | 14 |

Sterile toothpicks were used to transfer the transconjugants onto BHI agar, which was supplemented with 25 mg/l kanamycin and 50 mg/l nalidixic acid. The agar plates were incubated for 20 h at 33°C. The cultures of the respective transconjugants produced in this manner were then propagated further for 24 h at 33°C in 10 ml BHI medium contained in 100 ml Erlenmeyer flasks with 3 baffles. An aliquot was taken from the liquid culture suitably diluted and plated (typically 100 to 200 µl) on BHI agar which was supplemented with 10% saccharose. The agar plates were incubated for 48 h at 33°C. The colonies growing on the saccharose containing agar plates were then examined for the phenotype kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined for integration of the desired DNA fragment by means of real-time PCR.

### 8. Glycerol stocks of E.coli and C. glutamicum strains

For long time storage of *E.coli-* and *C. glutamicum* strains glycerol stocks were prepared. Selected *E. coli* clones were cultivated in 10 ml LB medium supplemented with 2 g/l glucose. Selected C. *glutamicum* clones were cultivated in two fold concentrated BHI medium supplemented with 2 g/l glucose. Cultures of plasmid containing *E*. *coli* strains were supplemented with 50 mg/l kanamycin. Cultures of plasmid containing *C. glutamicum* strains were supplemented with 25 mg/l kanamycin. The medium was contained in 100 ml Erlenmeyer flasks with 3 baffles. It was inoculated with a loop of cells taken from a colony and the culture incubated for about 18 h at 37°C and 200 rpm in the case of *E*. *coli* and 33°C and 200 rpm in the case of *C. glutamicum.* After said incubation period 1.2 ml 85% (v/v) sterile glycerol were added to the culture. The obtained glycerol containing cell suspension was then aliquoted in 2 ml portions and stored at -80°C.

### 9. Cultivation system according to Wouter Duetz (WDS)

The millilitre-scale cultivation system according to Duetz (Trends Microbiol. 2007; 15(10):469-75) was used to investigate the performance of the *C. glutamicum* strains constructed. For this purpose, 24-deepwell microplates (24 well WDS plates) from EnzyScreen BV (Heemstede, Netherlands; Cat. no. CR1424), filled with 2.5 mL medium were used.

Precultures of the strains were done in 10 ml two fold concentrated BHI medium. The medium was contained in a 100 ml Erlenmeyer flask with 3 baffles. It was inoculated with 100 µl of a glycerol stock culture and the culture incubated for 24 h at 33°C and 200 rpm.

After said incubation period the optical densities OD600 of the precultures were determined.

The main cultures were done by inoculating the 2.5 ml medium containing wells of the 24 Well WDS-Plate with an aliquot of the preculture to give an optical density OD600 of 0.1.

As medium for the main culture CGXII medium described by Keilhauer et al. (J. Bacteriol. 1993 Sep; 175(17): 5595-5603) was used. For convenience the composition of the CGXII medium is shown in table 3.

**Table 3: Composition of Keilhauer's CGXII medium.**

| **Components** | **Concentration (g/l)** |
|---|---|
| MOPS (3-(N-Morpholino)propanesulfonic acid) | 42 |
| (NH₄)₂SO₄ | 20 |
| Urea | 5 |
| KH₂PO₄ | 1 |
| K₂HPO₄ | 1 |
| MgSO₄ · 7 H₂O | 0.25 |
| CaCl₂ | 0.01 |
| FeSO₄ · 7 H₂O | 0.01 |
| MnSO₄ H₂O | 0.01 |
| ZnSO₄ · 7 H₂O | 0.001 |
| CuSO₄ · 5 H₂O | 0.0002 |
| NiCl₂ 6 H₂O | 0.00002 |
| Biotin (sterile-filtered) | 0.0002 |
| Protocatechuic acid (sterile-filtered) | 0.03 |
| Carbon source (sterile-filtered) | as needed |
| adjust the pH to 7 with NaOH | |

These main cultures were incubated for approximately 45 h at 33 °C and 300 rpm in an Infors HT Multitron standard incubator shaker from Infors GmbH (Bottmingen, Switzerland) until complete consumption of glucose.

The glucose concentration in the suspension was analysed with the blood glucose-meter OneTouch Vita® from LifeScan (Johnson & Johnson Medical GmbH, Neuss, Germany).

After cultivation the culture suspensions were transferred to a deep well microplate. A part of the culture suspension was suitably diluted to measure the OD600. Another part of the culture was centrifuged and the concentration of L-amino acids, in particular L-lysine, and residual glucose were analysed in the supernatant.

### 10. Amino acid analyser

The concentration of L-lysine and other L-amino acids, e.g. L-valine, in the culture supernatants was determined by ion exchange chromatography using a SYKAM S433 amino acid analyser from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aquous solution containing in 20 l 263 g trisodium citrate, 120 g citric acid, 1100 ml methanol, 100 ml 37 % HCI and 2 ml octanoic acid (final pH 3.5) was used. As buffer B an aquous solution containing in 20 I 392 g trisodium citrate, 100 g boric acid and 2 ml octanoic acid (final pH 10.2) was used. The free amino acids were coloured with ninhydrin through post-column derivatization and detected photometrically at 570 nm.

### 11. Glucose determination with continuous flow system (CFS)

A SANplus multi-channel continuous flow analyser from SKALAR analytic GmbH (Erkelenz, Germany) was used to determine the concentration of glucose in the supernatant. Glucose was detected with a coupled-enzyme assay (Hexokinase/ Glucose-6-Phosphate-Dehydrogenase) via NADH formation.

### B) EXPERIMENTAL RESULTS

### Example 1

Sequence of the *nusG* gene of *C. glutamicum* strain DM1933

Strain DM1933 is an L-lysine producer described by Blombach et al. (Applied and Environmental Microbiology 75(2), 419-427, 2009). It is deposited according to the Budapest treaty at the DSMZ under accession number DSM25442.

The nucleotide sequence of the chromosome of strain DM1933 was determined by Illumina whole-genome sequencing technology (Illumina Inc., San Diego, CA, US). See e.g. Benjak et al. (2015) Whole-Genome Sequencing for Comparative Genomics and De Novo Genome Assembly. In: Parish T., Roberts D. (eds) Mycobacteria Protocols. Methods in Molecular Biology, Vol 1285. Humana Press, NY, US) and Bennet, S. (Pharmacogenomics 5(4), 433-438, 2004).

It was found that the nucleotide sequence of the *nusG* coding sequence of strain DM1933 including the nucleotide sequence upstream and downstream thereof is identical to that of ATCC13032 shown in SEQ ID NO:1.

DM1933 contains in its chromosome a variant of the aspartokinase gene encoding a feedback resistant aspartokinase polypeptide. Said feedback resistant aspartokinase polypeptide has the amino acid sequence of SEQ ID NO:8 of the sequence listing, wherein the amino acid threonine (Thr) at position 311 of the amino acid sequence is replaced by isoleucine (Ile). In US 7,338,790 the abbreviation "lysC T3111" is used to indicate said exchange. Blombach et al. use the abbreviation "lysC(T311I)".

### Example 2

Construction of plasmid pK18mobsacB_nusG_N210D

Plasmid pK18mobsacB_nusG_N210D was constructed to enable incorporation of the mutation causing the amino acid exchange N210D into the nucleotide sequence of the *nusG* coding sequence of strain DM1933. The plasmid is based on the mobilizable vector pK18mobsacB described by Schafer et al. (Gene 145, 69-73, 1994). For the construction of pK18mobsacB_nusG_N210D the nusG_N210D-sequence shown in SEQ ID NO:9 was synthetized and subcloned into pK18mobsacB by GeneArt (ThermoFisher Scientific (Waltham, USA)).

To assemble the plasmid pK18mobsacB_nusG_N210D the two polynucleotides i.e. the vector pK18mobsacB cut with *XbaI* and the synthetized and with XbaI digested polynucleotide nusG_N210D were ligated and transformed in *E.coli* by GeneArt (ThermoFisher Scientific (Waltham, USA)).

### Example 3

Construction of strain DM1933_nusG_N210D

The plasmid pK18mobsacB_nusG_N210D obtained in example 2 was used to incorporate the mutation (see position 1002 of SEQ ID NO:1) leading to the amino acid exchange N210D (see nucleotide positions 1002-1004 of SEQ ID NO:3 or nucleotide position 805-807 of SEQ ID NO:9) into the chromosome of the L-lysine producer DM1933.

Chemically competent cells of *E. coli* strain S17-1 were transformed with plasmid DNA of pK18mobsacB_nusG_N210D. The modified conjugation method of Schafer et al. (Journal of Bacteriology 172, 1663 - 1666, 1990) as described in materials and methods was used for conjugal transfer into the strain DM1933 and for selection of transconjugant clones by virtue of their saccharose resistance and kanamycin sensitivity phenotype.

Transconjugant clones were analyzed by real-time PCR using the Type-it Kit and the primers N210D_for and N210D_rev for PCR amplification and NCgl0458_N210D_C as acceptor probe and NCgl0458_N210D_A as donor probe for melting curve analysis (table 4). Said primers and probes are also shown in SEQ ID NO's: 11, 12, 13 and 14 of the sequence listing.

**Table 4: List of primers and probes used for real-time PCR.**

| name | sequence |
|---|---|
| N210D_for | ACCTTGACATGCGTGCTCAG |
| N210D_rev | CATAGCCACAACCTGCTCAC |
| NCgl0458_N210D_C¹ | TGCCCTCGTCACCCACAAAG |
| NCgl0458_N210D_A² | AACTTCGCAACATCGCGGTGCTTCACAGGAGTTGC |

| | |
|---|---|
| ¹ acceptor probe labelled with LC-Red640 at the 5'-end and phosphorylated at the 3'-end ² donor probe labelled with fluorescein at the 3'-end | |

One of the transconjugant clones thus characterized was called DM1933_nusG_N210D. A glycerol stock culture of the transconjugant clone was prepared and used as starting material for further investigations.

Thus the *nusG* gene of strain DM1933 was mutated with the effect that the amino acid asparagine at position 210 of the amino acid sequence of the encoded NusG polypeptide was replaced by aspartic acid.

### Example 4

L-lysine production by strain DM1933_nusG_N210D

Strains DM1933 (reference) and DM1933_nusG_N210D obtained in example 3 were analyzed for their ability to produce L-lysine from glucose by batch cultivation using the cultivation system according to Wouter Duetz.

As medium CGXII containing 20 g/l glucose as carbon source was used. The cultures were incubated for 45 h until complete consumption of glucose as confirmed by glucose analysis using blood glucose-meter and the concentrations of L-lysine and the optical density OD660 were determined. The result of the experiment is presented in table 5.

**Table 5: L-lysine production by strain DM1933_nusG_N210D.**

| strain | L-lysine¹ (g/l) | OD660 |
|---|---|---|
| DM1933 | 3.7 | 9.5 |
| DM1933_nusG_N210D | 4.1 | 9.3 |

| | | |
|---|---|---|
| ¹ as L-lysine x HCL | | |

The experiment shows that L-lysine production was increased in strain DM1933_nusG_N210D as compared to the parent strain DM1933.

## Claims

1. A method for the fermentative production of L-lysine comprising the steps of
a) providing a bacterium of the species *Corynebacterium glutamicum* having the ability to excrete L-lysine containing in its chromosome a polynucleotide encoding a polypeptide having the activity of a transcriptional factor and comprising the amino acid sequence of SEQ ID NO:2, wherein the amino acid asparagine at position 210 is substituted by a different proteinogenic amino acid,
b) cultivating the bacterium in a suitable medium under suitable conditions, and
c) accumulating said L-lysine in the medium to form an L-lysine containing fermentation broth.

2. The method as claimed in claim 1, wherein in the bacterium provided said amino acid at position 210 of the amino acid sequence of SEQ ID NO:2 is aspartic acid or glutamic acid.

3. The method as claimed in claim 2, wherein in the bacterium provided said amino acid at position 210 of the amino acid sequence of SEQ ID NO:2 is aspartic acid.

4. The method as claimed in claim 3, wherein in the bacterium provided the polynucleotide encoding said amino acid sequence comprises the nucleotide sequence of positions 375 to 1328 of SEQ ID NO:1 the nucleobases at positions 1002 to 1004 being gac or gat.

5. The method as claimed in claim 3, wherein in the bacterium provided the polynucleotide encoding said amino acid sequence comprises the nucleotide sequence of positions 375 to 1331 of SEQ ID NO:1 the nucleobases at positions 1002 to 1004 being gac or gat.

6. The method as claimed in claim 3, wherein in the bacterium provided the polynucleotide encoding said amino acid sequence comprises the nucleotide sequence of positions 114 to 1331 of SEQ ID NO:1 the nucleobases at positions 1002 to 1004 being gac or gat.

7. The method as claimed in claim 3, wherein in the bacterium provided the polynucleotide encoding said amino acid sequence comprises the nucleotide sequence of positions 114 to 1402 of SEQ ID NO:1 the nucleobases at positions 1002 to 1004 being gac or gat.

8. The method as claimed in claim 3, wherein in the bacterium provided the polynucleotide encoding said amino acid sequence comprises the nucleotide sequence of positions 375 to 1328 of SEQ ID NO:5.

9. The method as claimed in claim 3, wherein in the bacterium provided the polynucleotide encoding said amino acid sequence comprises the nucleotide sequence of positions 375 to 1331 of SEQ ID NO:5.

10. The method as claimed in claim 3, wherein in the bacterium provided the polynucleotide encoding said amino acid sequence comprises the nucleotide sequence of positions 114 to 1331 of SEQ ID NO:5.

11. The method as claimed in claim 3, wherein in the bacterium provided the polynucleotide encoding said amino acid sequence comprises the nucleotide sequence of positions 114 to 1402 of SEQ ID NO:5.


SEQUENCE LISTING

<110>  Evonik Degussa GmbH

<120>  Process for the fermentative production of l-lysine

<130>  file: 201800170

<160>  14

<170>  PatentIn version 3.5

<210>  1
<211>  2001
<212>  DNA
<213>  Corynebacterium glutamicum ATCC13032


<220>
<221>  misc_feature
<222>  (114)..(114)
<223>  nuclebase guanine at position 114

<220>
<221>  misc_feature
<222>  (148)..(153)
<223>  - 10 region according to Pfeifer-Sancar et al. (2013)

<220>
<221>  misc_feature
<222>  (161)..(161)
<223>  transcriptional start site according to Pfeifer-Sancar et al.
       (2013)

<220>
<221>  CDS
<222>  (375)..(1328)

<220>
<221>  misc_feature
<222>  (1002)..(1004)
<223>  aac codon for asparagine

<220>
<221>  misc_feature
<222>  (1002)..(1002)
<223>  nucleobase adenine

<220>
<221>  misc_feature
<222>  (1329)..(1331)
<223>  tag stop codon

<220>
<221>  misc_feature
<222>  (1332)..(1334)
<223>  tag stop codon

<220>
<221>  misc_feature
<222>  (1350)..(1402)
<223>  nucleotide sequence for a stem-and-loop structure (see Fig. 2 of
       Barreiro et al., 2001)

<400> 1
aagtccgtaa ggttatttgg cctactgcgc gccagatggt cacgtacacc cttgtcgttt 60

tgggattctt gattgttttg accgctttgg tgtctggtgt ggatttccta gctggtcttg 120

gagttgagaa gattctgact ccgtaggtag gatgtgtaac atcttttttg aaaagtcccg 180

ctggttccct ggaggagccg gcgggataat ttttgcccag gtggttggtt tggggcagcg 240

gttgcaattg gatgtaattg gttgtttgtc gcagtagcat gggacgaaag ctgttagata 300

gcatgttgca tccctgcgtt ggctgattat cgctgggttt tagggtcgat agataggttg 360

ggagaacacg catt atg agc gat gag aac att aac gag ttt gag cag gac 410
Met Ser Asp Glu Asn Ile Asn Glu Phe Glu Gln Asp
1               5                   10

gag gat ctg aac ttc ggc gcg agc ttt agt gat gaa ttc gca gat gac 458
Glu Asp Leu Asn Phe Gly Ala Ser Phe Ser Asp Glu Phe Ala Asp Asp
        15                  20                  25

gat ttc gat gca gaa gca gac gta gaa gca gat gct gct gca gag gcc 506
Asp Phe Asp Ala Glu Ala Asp Val Glu Ala Asp Ala Ala Ala Glu Ala
    30                  35                  40

tct gcc ctg gaa gct gag cag gat ctg gaa gaa gag acc cta gat gct 554
Ser Ala Leu Glu Ala Glu Gln Asp Leu Glu Glu Glu Thr Leu Asp Ala
45                  50                  55                  60

cca gaa gaa gcc gca gaa gaa gct cct gct gct gca gag tcc gaa gct 602
Pro Glu Glu Ala Ala Glu Glu Ala Pro Ala Ala Ala Glu Ser Glu Ala
                65                  70                  75

cca gta gaa gag gac gaa gag gct gac agc ctt gct cag gcg gct gct 650
Pro Val Glu Glu Asp Glu Glu Ala Asp Ser Leu Ala Gln Ala Ala Ala
            80                  85                  90

gca ctt ggt gac acc gat gag cag gac gcg gat gca gag tac aag gct 698
Ala Leu Gly Asp Thr Asp Glu Gln Asp Ala Asp Ala Glu Tyr Lys Ala
        95                  100                 105

cgt ctg cgt aag ttc act cgt gag ctg aag aag cag cct ggt gtt tgg 746
Arg Leu Arg Lys Phe Thr Arg Glu Leu Lys Lys Gln Pro Gly Val Trp
    110                 115                 120

tac atc att cag tgc tac tcc ggc tac gag aac aag gtg aag gcg aac 794
Tyr Ile Ile Gln Cys Tyr Ser Gly Tyr Glu Asn Lys Val Lys Ala Asn
125                 130                 135                 140

ctt gac atg cgt gct cag acc ctt gag gtt gag gat gac atc ttt gag 842
Leu Asp Met Arg Ala Gln Thr Leu Glu Val Glu Asp Asp Ile Phe Glu
                145                 150                 155

gtt gtt gtt cct atc gag cag gtc act gag atc cgt gat ggt aag cgc 890
Val Val Val Pro Ile Glu Gln Val Thr Glu Ile Arg Asp Gly Lys Arg
            160                 165                 170

aag ctg gtt aag cgt aag ttg ctg ccg ggc tac gtt ttg gtc cgc atg 938
Lys Leu Val Lys Arg Lys Leu Leu Pro Gly Tyr Val Leu Val Arg Met
        175                 180                 185

gac atg aat gac cgc gtg tgg tct gtt gtt cgc gat aca cct ggt gtg 986

Asp Met Asn Asp Arg Val Trp Ser Val Val Arg Asp Thr Pro Gly Val
    190                 195                 200

acc agc ttt gtg ggt aac gag ggc aat gca act cct gtg aag cac cgc      1034
Thr Ser Phe Val Gly Asn Glu Gly Asn Ala Thr Pro Val Lys His Arg
205                 210                 215                 220

gat gtt gcg aag ttc ttg atg cct cag gag cag gct gtt gtc acc ggt      1082
Asp Val Ala Lys Phe Leu Met Pro Gln Glu Gln Ala Val Val Thr Gly
                225                 230                 235

gag gct gct gct gcg gct gcc gag ggt gag cag gtt gtg gct atg cct      1130
Glu Ala Ala Ala Ala Ala Ala Glu Gly Glu Gln Val Val Ala Met Pro
            240                 245                 250

acc gat acc aag aag cct cag gtt gct gtg gac ttc act gtt ggt gag      1178
Thr Asp Thr Lys Lys Pro Gln Val Ala Val Asp Phe Thr Val Gly Glu
        255                 260                 265

gct gtg acc att ctg act ggt gct ttc gct tct gtt tct gca acg att      1226
Ala Val Thr Ile Leu Thr Gly Ala Phe Ala Ser Val Ser Ala Thr Ile
    270                 275                 280

tct tct atc gat cct gag ctg cag aag ctg gaa gtt ttg gtg tcc atc      1274
Ser Ser Ile Asp Pro Glu Leu Gln Lys Leu Glu Val Leu Val Ser Ile
285                 290                 295                 300

ttt ggt cgt gaa act cct gtt gat ctc agc ttc gac cag gtt gag aag      1322
Phe Gly Arg Glu Thr Pro Val Asp Leu Ser Phe Asp Gln Val Glu Lys
                305                 310                 315

gtt agc tagtagctaa actgcaccac ttaccccgca tttcctaggc cacatataag      1378
Val Ser

ggctttggtg atgcggggtt ttgcgtgtag ggtagacaat cgcgtgtttt ttaagcatgc      1438

tcaaaatcat tcatccccgg tggcccggtt acgtaaagat cagcaaagat gatcaactaa      1498

agcgatcatc tgaagttgta gcgggaccga gcatccggac ggttactagt ggggtttcat      1558

cgtcccagtt gtggccggta acaaggaagc aggtttaacg atggctccta agaagaagaa      1618

gaaggtcact ggcctcatca agctccagat ccaggcagga caggcaaacc ctgctcctcc      1678

agttggccca gcacttggtg ctcacggcgt caacatcatg gaattctgca aggcttacaa      1738

cgctgcgact gaaaaccagc gcggcaacgt tgttcctgtt gagatcaccg tttacgaaga      1798

ccgttcattc gacttcaagc tgaagactcc tccagctgca aagcttcttc tgaaggctgc      1858

tggcctgcag aagggctccg gcgttcctca cacccagaag gtcggcaagg tttccatggc      1918

tcaggttcgt gagatcgctg agaccaagaa ggaagacctg aacgctcgcg atatcgacgc      1978

tgctgcgaag atcatcgctg gta                                            2001

<210>  2
<211>  318
<212>  PRT
<213>  Corynebacterium glutamicum ATCC13032

<400>  2
   Met Ser Asp Glu Asn Ile Asn Glu Phe Glu Gln Asp Glu Asp Leu Asn
   1               5                   10                  15
   Phe Gly Ala Ser Phe Ser Asp Glu Phe Ala Asp Asp Asp Phe Asp Ala
               20                  25                  30
   Glu Ala Asp Val Glu Ala Asp Ala Ala Ala Glu Ala Ser Ala Leu Glu
           35                  40                  45
   Ala Glu Gln Asp Leu Glu Glu Glu Thr Leu Asp Ala Pro Glu Glu Ala
       50                  55                  60
   Ala Glu Glu Ala Pro Ala Ala Ala Glu Ser Glu Ala Pro Val Glu Glu
   65                  70                  75                  80
   Asp Glu Glu Ala Asp Ser Leu Ala Gln Ala Ala Ala Ala Leu Gly Asp
                   85                  90                  95
   Thr Asp Glu Gln Asp Ala Asp Ala Glu Tyr Lys Ala Arg Leu Arg Lys
               100                 105                 110
   Phe Thr Arg Glu Leu Lys Lys Gln Pro Gly Val Trp Tyr Ile Ile Gln
           115                 120                 125
   Cys Tyr Ser Gly Tyr Glu Asn Lys Val Lys Ala Asn Leu Asp Met Arg
       130                 135                 140
   Ala Gln Thr Leu Glu Val Glu Asp Asp Ile Phe Glu Val Val Val Pro
   145                 150                 155                 160
   Ile Glu Gln Val Thr Glu Ile Arg Asp Gly Lys Arg Lys Leu Val Lys
                   165                 170                 175
   Arg Lys Leu Leu Pro Gly Tyr Val Leu Val Arg Met Asp Met Asn Asp
               180                 185                 190
   Arg Val Trp Ser Val Val Arg Asp Thr Pro Gly Val Thr Ser Phe Val
           195                 200                 205
   Gly Asn Glu Gly Asn Ala Thr Pro Val Lys His Arg Asp Val Ala Lys
       210                 215                 220
   Phe Leu Met Pro Gln Glu Gln Ala Val Val Thr Gly Glu Ala Ala Ala
   225                 230                 235                 240
   Ala Ala Ala Glu Gly Glu Gln Val Val Ala Met Pro Thr Asp Thr Lys
   245                 250                 255
   Lys Pro Gln Val Ala Val Asp Phe Thr Val Gly Glu Ala Val Thr Ile
               260                 265                 270
   Leu Thr Gly Ala Phe Ala Ser Val Ser Ala Thr Ile Ser Ser Ile Asp
           275                 280                 285
   Pro Glu Leu Gln Lys Leu Glu Val Leu Val Ser Ile Phe Gly Arg Glu
       290                 295                 300
   Thr Pro Val Asp Leu Ser Phe Asp Gln Val Glu Lys Val Ser
   305                 310                 315

<210>  3
<211>  2001
<212>  DNA
<213>  Corynebacterium glutamicum

<220>
<221>  misc_feature
<222>  (114)..(114)
<223>  nucleobase guanine at position 114

<220>
<221>  misc_feature
<222>  (148)..(153)
<223>  - 10 region according to Pfeifer-Sancar et al. (2013)

<220>
<221>  misc_feature
<222>  (161)..(161)
<223>  transcriptional start site according to Pfeifer-Sancar et al.
       (2013)

<220>
<221>  CDS
<222>  (375)..(1328)

<220>
<221>  misc_feature
<222>  (1002)..(1004)
<223>  gac codon for aspartic acid

<220>
<221>  misc_feature
<222>  (1002)..(1002)
<223>  nucleobase guanine

<220>
<221>  misc_feature
<222>  (1329)..(1331)
<223>  tag stop codon

<220>
<221>  misc_feature
<222>  (1332)..(1334)

<223>  tag stop codon

<220>
<221>  misc_feature
<222>  (1350)..(1402)
<223>  nucleotide sequence for a stem-and-loop structure (see Fig. 2 of
       Barreiro et al., 2001)

<400>  3
aagtccgtaa ggttatttgg cctactgcgc gccagatggt cacgtacacc cttgtcgttt      60

tgggattctt gattgttttg accgctttgg tgtctggtgt ggatttccta gctggtcttg     120

gagttgagaa gattctgact ccgtaggtag gatgtgtaac atcttttttg aaaagtcccg     180

ctggttccct ggaggagccg gcgggataat ttttgcccag gtggttggtt tggggcagcg     240

gttgcaattg gatgtaattg gttgtttgtc gcagtagcat gggacgaaag ctgttagata     300

gcatgttgca tccctgcgtt ggctgattat cgctgggttt tagggtcgat agataggttg     360

ggagaacacg catt atg agc gat gag aac att aac gag ttt gag cag gac       410
                Met Ser Asp Glu Asn Ile Asn Glu Phe Glu Gln Asp
                1               5                   10

gag gat ctg aac ttc ggc gcg agc ttt agt gat gaa ttc gca gat gac       458
Glu Asp Leu Asn Phe Gly Ala Ser Phe Ser Asp Glu Phe Ala Asp Asp
        15                  20                  25

gat ttc gat gca gaa gca gac gta gaa gca gat gct gct gca gag gcc       506
Asp Phe Asp Ala Glu Ala Asp Val Glu Ala Asp Ala Ala Ala Glu Ala
    30                  35                  40

tct gcc ctg gaa gct gag cag gat ctg gaa gaa gag acc cta gat gct       554
Ser Ala Leu Glu Ala Glu Gln Asp Leu Glu Glu Glu Thr Leu Asp Ala
45                  50                  55                  60

cca gaa gaa gcc gca gaa gaa gct cct gct gct gca gag tcc gaa gct       602
Pro Glu Glu Ala Ala Glu Glu Ala Pro Ala Ala Ala Glu Ser Glu Ala
                65                  70                  75

cca gta gaa gag gac gaa gag gct gac agc ctt gct cag gcg gct gct       650
Pro Val Glu Glu Asp Glu Glu Ala Asp Ser Leu Ala Gln Ala Ala Ala
            80                  85                  90

gca ctt ggt gac acc gat gag cag gac gcg gat gca gag tac aag gct       698
Ala Leu Gly Asp Thr Asp Glu Gln Asp Ala Asp Ala Glu Tyr Lys Ala
        95                  100                 105

cgt ctg cgt aag ttc act cgt gag ctg aag aag cag cct ggt gtt tgg       746
Arg Leu Arg Lys Phe Thr Arg Glu Leu Lys Lys Gln Pro Gly Val Trp
    110                 115                 120

tac atc att cag tgc tac tcc ggc tac gag aac aag gtg aag gcg aac       794
Tyr Ile Ile Gln Cys Tyr Ser Gly Tyr Glu Asn Lys Val Lys Ala Asn
125                 130                 135                 140

ctt gac atg cgt gct cag acc ctt gag gtt gag gat gac atc ttt gag       842
Leu Asp Met Arg Ala Gln Thr Leu Glu Val Glu Asp Asp Ile Phe Glu
                145                 150                 155

gtt gtt gtt cct atc gag cag gtc act gag atc cgt gat ggt aag cgc       890
Val Val Val Pro Ile Glu Gln Val Thr Glu Ile Arg Asp Gly Lys Arg

160                 165                 170

aag ctg gtt aag cgt aag ttg ctg ccg ggc tac gtt ttg gtc cgc atg      938
Lys Leu Val Lys Arg Lys Leu Leu Pro Gly Tyr Val Leu Val Arg Met
        175                 180                 185

gac atg aat gac cgc gtg tgg tct gtt gtt cgc gat aca cct ggt gtg      986
Asp Met Asn Asp Arg Val Trp Ser Val Val Arg Asp Thr Pro Gly Val
    190                 195                 200

acc agc ttt gtg ggt gac gag ggc aat gca act cct gtg aag cac cgc      1034
Thr Ser Phe Val Gly Asp Glu Gly Asn Ala Thr Pro Val Lys His Arg
205                 210                 215                 220

gat gtt gcg aag ttc ttg atg cct cag gag cag gct gtt gtc acc ggt      1082
Asp Val Ala Lys Phe Leu Met Pro Gln Glu Gln Ala Val Val Thr Gly
                225                 230                 235

gag gct gct gct gcg gct gcc gag ggt gag cag gtt gtg gct atg cct      1130
Glu Ala Ala Ala Ala Ala Ala Glu Gly Glu Gln Val Val Ala Met Pro
            240                 245                 250

acc gat acc aag aag cct cag gtt gct gtg gac ttc act gtt ggt gag      1178
Thr Asp Thr Lys Lys Pro Gln Val Ala Val Asp Phe Thr Val Gly Glu
        255                 260                 265

gct gtg acc att ctg act ggt gct ttc gct tct gtt tct gca acg att      1226
Ala Val Thr Ile Leu Thr Gly Ala Phe Ala Ser Val Ser Ala Thr Ile
    270                 275                 280

tct tct atc gat cct gag ctg cag aag ctg gaa gtt ttg gtg tcc atc      1274
Ser Ser Ile Asp Pro Glu Leu Gln Lys Leu Glu Val Leu Val Ser Ile
285                 290                 295                 300

ttt ggt cgt gaa act cct gtt gat ctc agc ttc gac cag gtt gag aag      1322
Phe Gly Arg Glu Thr Pro Val Asp Leu Ser Phe Asp Gln Val Glu Lys
                305                 310                 315

gtt agc tagtagctaa actgcaccac ttaccccgca tttcctaggc cacatataag      1378
Val Ser

ggctttggtg atgcggggtt ttgcgtgtag ggtagacaat cgcgtgtttt ttaagcatgc      1438

tcaaaatcat tcatccccgg tggcccggtt acgtaaagat cagcaaagat gatcaactaa      1498

agcgatcatc tgaagttgta gcgggaccga gcatccggac ggttactagt ggggtttcat      1558

cgtcccagtt gtggccggta acaaggaagc aggtttaacg atggctccta agaagaagaa      1618

gaaggtcact ggcctcatca agctccagat ccaggcagga caggcaaacc ctgctcctcc      1678

agttggccca gcacttggtg ctcacggcgt caacatcatg gaattctgca aggcttacaa      1738

cgctgcgact gaaaaccagc gcggcaacgt tgttcctgtt gagatcaccg tttacgaaga      1798

ccgttcattc gacttcaagc tgaagactcc tccagctgca aagcttcttc tgaaggctgc      1858

tggcctgcag aagggctccg gcgttcctca cacccagaag gtcggcaagg tttccatggc      1918

tcaggttcgt gagatcgctg agaccaagaa ggaagacctg aacgctcgcg atatcgacgc      1978

tgctgcgaag atcatcgctg gta      2001

<210>  4
<211>  318
<212>  PRT
<213>  Corynebacterium glutamicum

<400>  4

Met Ser Asp Glu Asn Ile Asn Glu Phe Glu Gln Asp Glu Asp Leu Asn
1               5                   10                  15

Phe Gly Ala Ser Phe Ser Asp Glu Phe Ala Asp Asp Asp Phe Asp Ala
            20                  25                  30

Glu Ala Asp Val Glu Ala Asp Ala Ala Ala Glu Ala Ser Ala Leu Glu
        35                  40                  45

Ala Glu Gln Asp Leu Glu Glu Glu Thr Leu Asp Ala Pro Glu Glu Ala
    50                  55                  60

Ala Glu Glu Ala Pro Ala Ala Ala Glu Ser Glu Ala Pro Val Glu Glu
65                  70                  75                  80

Asp Glu Glu Ala Asp Ser Leu Ala Gln Ala Ala Ala Ala Leu Gly Asp
                85                  90                  95

Thr Asp Glu Gln Asp Ala Asp Ala Glu Tyr Lys Ala Arg Leu Arg Lys
            100                 105                 110

Phe Thr Arg Glu Leu Lys Lys Gln Pro Gly Val Trp Tyr Ile Ile Gln
        115                 120                 125

Cys Tyr Ser Gly Tyr Glu Asn Lys Val Lys Ala Asn Leu Asp Met Arg
    130                 135                 140

Ala Gln Thr Leu Glu Val Glu Asp Asp Ile Phe Glu Val Val Val Pro
145                 150                 155                 160

Ile Glu Gln Val Thr Glu Ile Arg Asp Gly Lys Arg Lys Leu Val Lys
                165                 170                 175

Arg Lys Leu Leu Pro Gly Tyr Val Leu Val Arg Met Asp Met Asn Asp
            180                 185                 190

Arg Val Trp Ser Val Val Arg Asp Thr Pro Gly Val Thr Ser Phe Val
        195                 200                 205

Gly Asp Glu Gly Asn Ala Thr Pro Val Lys His Arg Asp Val Ala Lys
    210                 215                 220

Phe Leu Met Pro Gln Glu Gln Ala Val Val Thr Gly Glu Ala Ala Ala
225                 230                 235                 240

Ala Ala Ala Glu Gly Glu Gln Val Val Ala Met Pro Thr Asp Thr Lys
                245                 250                 255

Lys Pro Gln Val Ala Val Asp Phe Thr Val Gly Glu Ala Val Thr Ile
            260                 265                 270

Leu Thr Gly Ala Phe Ala Ser Val Ser Ala Thr Ile Ser Ser Ile Asp
        275                 280                 285

Pro Glu Leu Gln Lys Leu Glu Val Leu Val Ser Ile Phe Gly Arg Glu
    290                 295                 300

Thr Pro Val Asp Leu Ser Phe Asp Gln Val Glu Lys Val Ser
305                 310                 315

<210>  5
<211>  2001
<212>  DNA
<213>  Corynebacterium glutamicum

<220>
<221>  misc_feature
<222>  (114)..(114)
<223>  nucleobase guanine at position 114

<220>
<221>  misc_feature
<222>  (148)..(153)
<223>  - 10 region according to Pfeifer-Sancar et al. (2013)

<220>
<221>  misc_feature
<222>  (161)..(161)
<223>  transcriptional start site according to Pfeifer-Sancar et al.
       (2013)

<220>
<221>  CDS
<222>  (375)..(1328)

<220>
<221>  misc_feature
<222>  (1002)..(1004)
<223>  gat codon for aspartic acid

<220>
<221>  misc_feature
<222>  (1002)..(1002)
<223>  nucleobase guanine

<220>

<221>  misc_feature
<222>  (1329)..(1331)
<223>  tag stop codon

<220>
<221>  misc_feature
<222>  (1332)..(1334)
<223>  tag stop codon

<220>
<221>  misc_feature
<222>  (1350)..(1402)
<223>  nucleotide sequence for a stem-and-loop structure (see Fig.2 of
       Barreiro et al., 2001)

<400>  5
aagtccgtaa ggttatttgg cctactgcgc gccagatggt cacgtacacc cttgtcgttt      60

tgggattctt gattgttttg accgctttgg tgtctggtgt ggatttccta gctggtcttg     120

gagttgagaa gattctgact ccgtaggtag gatgtgtaac atcttttttg aaaagtcccg      180

ctggttccct ggaggagccg gcgggataat ttttgcccag gtggttggtt tggggcagcg      240

gttgcaattg gatgtaattg gttgtttgtc gcagtagcat gggacgaaag ctgttagata     300

gcatgttgca tccctgcgtt ggctgattat cgctgggttt tagggtcgat agataggttg     360

ggagaacacg catt atg agc gat gag aac att aac gag ttt gag cag gac       410
                Met Ser Asp Glu Asn Ile Asn Glu Phe Glu Gln Asp
                1               5                   10

gag gat ctg aac ttc ggc gcg agc ttt agt gat gaa ttc gca gat gac       458
Glu Asp Leu Asn Phe Gly Ala Ser Phe Ser Asp Glu Phe Ala Asp Asp
        15                  20                  25

gat ttc gat gca gaa gca gac gta gaa gca gat gct gct gca gag gcc       506
Asp Phe Asp Ala Glu Ala Asp Val Glu Ala Asp Ala Ala Ala Glu Ala
    30                  35                  40

tct gcc ctg gaa gct gag cag gat ctg gaa gaa gag acc cta gat gct       554
Ser Ala Leu Glu Ala Glu Gln Asp Leu Glu Glu Glu Thr Leu Asp Ala
45                  50                  55                  60

cca gaa gaa gcc gca gaa gaa gct cct gct gct gca gag tcc gaa gct       602
Pro Glu Glu Ala Ala Glu Glu Ala Pro Ala Ala Ala Glu Ser Glu Ala
                65                  70                  75

cca gta gaa gag gac gaa gag gct gac agc ctt gct cag gcg gct gct       650
Pro Val Glu Glu Asp Glu Glu Ala Asp Ser Leu Ala Gln Ala Ala Ala
            80                  85                  90

gca ctt ggt gac acc gat gag cag gac gcg gat gca gag tac aag gct       698
Ala Leu Gly Asp Thr Asp Glu Gln Asp Ala Asp Ala Glu Tyr Lys Ala
        95                  100                 105

cgt ctg cgt aag ttc act cgt gag ctg aag aag cag cct ggt gtt tgg       746
Arg Leu Arg Lys Phe Thr Arg Glu Leu Lys Lys Gln Pro Gly Val Trp
    110                 115                 120

tac atc att cag tgc tac tcc ggc tac gag aac aag gtg aag gcg aac       794
Tyr Ile Ile Gln Cys Tyr Ser Gly Tyr Glu Asn Lys Val Lys Ala Asn
125                 130                 135                 140

ctt gac atg cgt gct cag acc ctt gag gtt gag gat gac atc ttt gag 842
Leu Asp Met Arg Ala Gln Thr Leu Glu Val Glu Asp Asp Ile Phe Glu
                145                 150                 155

gtt gtt gtt cct atc gag cag gtc act gag atc cgt gat ggt aag cgc 890
Val Val Val Pro Ile Glu Gln Val Thr Glu Ile Arg Asp Gly Lys Arg
            160                 165                 170

aag ctg gtt aag cgt aag ttg ctg ccg ggc tac gtt ttg gtc cgc atg 938
Lys Leu Val Lys Arg Lys Leu Leu Pro Gly Tyr Val Leu Val Arg Met
        175                 180                 185

gac atg aat gac cgc gtg tgg tct gtt gtt cgc gat aca cct ggt gtg 986
Asp Met Asn Asp Arg Val Trp Ser Val Val Arg Asp Thr Pro Gly Val
    190                 195                 200

acc agc ttc gtc ggc gat gag ggc aat gca act cct gtg aag cac cgc 1034
Thr Ser Phe Val Gly Asp Glu Gly Asn Ala Thr Pro Val Lys His Arg
205                 210                 215                 220

gat gtt gcg aag ttc ttg atg cct cag gag cag gct gtt gtc acc ggt 1082
Asp Val Ala Lys Phe Leu Met Pro Gln Glu Gln Ala Val Val Thr Gly
                225                 230                 235

gag gct gct gct gcg gct gcc gag ggt gag cag gtt gtg gct atg cct 1130
Glu Ala Ala Ala Ala Ala Ala Glu Gly Glu Gln Val Val Ala Met Pro
            240                 245                 250

acc gat acc aag aag cct cag gtt gct gtg gac ttc act gtt ggt gag 1178
Thr Asp Thr Lys Lys Pro Gln Val Ala Val Asp Phe Thr Val Gly Glu
        255                 260                 265

gct gtg acc att ctg act ggt gct ttc gct tct gtt tct gca acg att 1226
Ala Val Thr Ile Leu Thr Gly Ala Phe Ala Ser Val Ser Ala Thr Ile
    270                 275                 280

tct tct atc gat cct gag ctg cag aag ctg gaa gtt ttg gtg tcc atc 1274
Ser Ser Ile Asp Pro Glu Leu Gln Lys Leu Glu Val Leu Val Ser Ile
285                 290                 295                 300

ttt ggt cgt gaa act cct gtt gat ctc agc ttc gac cag gtt gag aag 1322
Phe Gly Arg Glu Thr Pro Val Asp Leu Ser Phe Asp Gln Val Glu Lys
                305                 310                 315

gtt agc tagtagctaa actgcaccac ttaccccgca tttcctaggc cacatataag 1378
Val Ser

ggctttggtg atgcggggtt ttgcgtgtag ggtagacaat cgcgtgtttt ttaagcatgc 1438

tcaaaatcat tcatccccgg tggcccggtt acgtaaagat cagcaaagat gatcaactaa 1498

agcgatcatc tgaagttgta gcgggaccga gcatccggac ggttactagt ggggtttcat 1558

cgtcccagtt gtggccggta acaaggaagc aggtttaacg atggctccta agaagaagaa 1618

gaaggtcact ggcctcatca agctccagat ccaggcagga caggcaaacc ctgctcctcc 1678

agttggccca gcacttggtg ctcacggcgt caacatcatg gaattctgca aggcttacaa 1738

cgctgcgact gaaaaccagc gcggcaacgt tgttcctgtt gagatcaccg tttacgaaga 1798

ccgttcattc gacttcaagc tgaagactcc tccagctgca aagcttcttc tgaaggctgc     1858

tggcctgcag aagggctccg gcgttcctca cacccagaag gtcggcaagg tttccatggc     1918

tcaggttcgt gagatcgctg agaccaagaa ggaagacctg aacgctcgcg atatcgacgc     1978

tgctgcgaag atcatcgctg gta                                             2001

<210>  6
<211>  318
<212>  PRT
<213>  Corynebacterium glutamicum

<400>  6

Met Ser Asp Glu Asn Ile Asn Glu Phe Glu Gln Asp Glu Asp Leu Asn
1               5                   10                  15

Phe Gly Ala Ser Phe Ser Asp Glu Phe Ala Asp Asp Asp Phe Asp Ala
            20                  25                  30

Glu Ala Asp Val Glu Ala Asp Ala Ala Ala Glu Ala Ser Ala Leu Glu
        35                  40                  45

Ala Glu Gln Asp Leu Glu Glu Glu Thr Leu Asp Ala Pro Glu Glu Ala
    50                  55                  60

Ala Glu Glu Ala Pro Ala Ala Ala Glu Ser Glu Ala Pro Val Glu Glu
65                  70                  75                  80

Asp Glu Glu Ala Asp Ser Leu Ala Gln Ala Ala Ala Ala Leu Gly Asp
                85                  90                  95

Thr Asp Glu Gln Asp Ala Asp Ala Glu Tyr Lys Ala Arg Leu Arg Lys
            100                 105                 110

Phe Thr Arg Glu Leu Lys Lys Gln Pro Gly Val Trp Tyr Ile Ile Gln
        115                 120                 125

Cys Tyr Ser Gly Tyr Glu Asn Lys Val Lys Ala Asn Leu Asp Met Arg
    130                 135                 140

Ala Gln Thr Leu Glu Val Glu Asp Asp Ile Phe Glu Val Val Val Pro
145                 150                 155                 160

Ile Glu Gln Val Thr Glu Ile Arg Asp Gly Lys Arg Lys Leu Val Lys
                165                 170                 175

Arg Lys Leu Leu Pro Gly Tyr Val Leu Val Arg Met Asp Met Asn Asp
            180                 185                 190

Arg Val Trp Ser Val Val Arg Asp Thr Pro Gly Val Thr Ser Phe Val
        195                 200                 205

Gly Asp Glu Gly Asn Ala Thr Pro Val Lys His Arg Asp Val Ala Lys
    210                 215                 220

Phe Leu Met Pro Gln Glu Gln Ala Val Val Thr Gly Glu Ala Ala Ala
225                 230                 235                 240

Ala Ala Ala Glu Gly Glu Gln Val Val Ala Met Pro Thr Asp Thr Lys
                245                 250                 255

Lys Pro Gln Val Ala Val Asp Phe Thr Val Gly Glu Ala Val Thr Ile
            260                 265                 270

Leu Thr Gly Ala Phe Ala Ser Val Ser Ala Thr Ile Ser Ser Ile Asp
        275                 280                 285

Pro Glu Leu Gln Lys Leu Glu Val Leu Val Ser Ile Phe Gly Arg Glu
    290                 295                 300

Thr Pro Val Asp Leu Ser Phe Asp Gln Val Glu Lys Val Ser
305                 310                 315

<210>  7
<211>  1266
<212>  DNA
<213>  Corynebacterium glutamicum ATCC13032

<220>
<221>  CDS
<222>  (1)..(1263)

<400>  7
gtg gcc ctg gtc gta cag aaa tat ggc ggt tcc tcg ctt gag agt gcg        48
Met Ala Leu Val Val Gln Lys Tyr Gly Gly Ser Ser Leu Glu Ser Ala
1               5                   10                  15

gaa cgc att aga aac gtc gct gaa cgg atc gtt gcc acc aag aag gct        96
Glu Arg Ile Arg Asn Val Ala Glu Arg Ile Val Ala Thr Lys Lys Ala
            20                  25                  30

gga aat gat gtc gtg gtt gtc tgc tcc gca atg gga gac acc acg gat       144
Gly Asn Asp Val Val Val Val Cys Ser Ala Met Gly Asp Thr Thr Asp
        35                  40                  45

gaa ctt cta gaa ctt gca gcg gca gtg aat ccc gtt ccg cca gct cgt       192
Glu Leu Leu Glu Leu Ala Ala Ala Val Asn Pro Val Pro Pro Ala Arg
    50                  55                  60

gaa atg gat atg ctc ctg act gct ggt gag cgt att tct aac gct ctc       240
Glu Met Asp Met Leu Leu Thr Ala Gly Glu Arg Ile Ser Asn Ala Leu
65                  70                  75                  80

gtc gcc atg gct att gag tcc ctt ggc gca gaa gcc caa tct ttc acg 288
Val Ala Met Ala Ile Glu Ser Leu Gly Ala Glu Ala Gln Ser Phe Thr
                85                  90                  95

ggc tct cag gct ggt gtg ctc acc acc gag cgc cac gga aac gca cgc 336
Gly Ser Gln Ala Gly Val Leu Thr Thr Glu Arg His Gly Asn Ala Arg
            100                 105                 110

att gtt gat gtc act cca ggt cgt gtg cgt gaa gca ctc gat gag ggc 384
Ile Val Asp Val Thr Pro Gly Arg Val Arg Glu Ala Leu Asp Glu Gly
        115                 120                 125

aag atc tgc att gtt gct ggt ttc cag ggt gtt aat aaa gaa acc cgc 432
Lys Ile Cys Ile Val Ala Gly Phe Gln Gly Val Asn Lys Glu Thr Arg
    130                 135                 140

gat gtc acc acg ttg ggt cgt ggt ggt tct gac acc act gca gtt gcg 480
Asp Val Thr Thr Leu Gly Arg Gly Gly Ser Asp Thr Thr Ala Val Ala
145                 150                 155                 160

ttg gca gct gct ttg aac gct gat gtg tgt gag att tac tcg gac gtt 528
Leu Ala Ala Ala Leu Asn Ala Asp Val Cys Glu Ile Tyr Ser Asp Val
                165                 170                 175

gac ggt gtg tat acc gct gac ccg cgc atc gtt cct aat gca cag aag 576
Asp Gly Val Tyr Thr Ala Asp Pro Arg Ile Val Pro Asn Ala Gln Lys
            180                 185                 190

ctg gaa aag ctc agc ttc gaa gaa atg ctg gaa ctt gct gct gtt ggc 624
Leu Glu Lys Leu Ser Phe Glu Glu Met Leu Glu Leu Ala Ala Val Gly
        195                 200                 205

tcc aag att ttg gtg ctg cgc agt gtt gaa tac gct cgt gca ttc aat 672
Ser Lys Ile Leu Val Leu Arg Ser Val Glu Tyr Ala Arg Ala Phe Asn
    210                 215                 220

gtg cca ctt cgc gta cgc tcg tct tat agt aat gat ccc ggc act ttg 720
Val Pro Leu Arg Val Arg Ser Ser Tyr Ser Asn Asp Pro Gly Thr Leu
225                 230                 235                 240

att gcc ggc tct atg gag gat att cct gtg gaa gaa gca gtc ctt acc 768
Ile Ala Gly Ser Met Glu Asp Ile Pro Val Glu Glu Ala Val Leu Thr
                245                 250                 255

ggt gtc gca acc gac aag tcc gaa gcc aaa gta acc gtt ctg ggt att 816
Gly Val Ala Thr Asp Lys Ser Glu Ala Lys Val Thr Val Leu Gly Ile
            260                 265                 270

tcc gat aag cca ggc gag gct gcg aag gtt ttc cgt gcg ttg gct gat 864
Ser Asp Lys Pro Gly Glu Ala Ala Lys Val Phe Arg Ala Leu Ala Asp
        275                 280                 285

gca gaa atc aac att gac atg gtt ctg cag aac gtc tct tct gta gaa 912
Ala Glu Ile Asn Ile Asp Met Val Leu Gln Asn Val Ser Ser Val Glu
    290                 295                 300

gac ggc acc acc gac atc acc ttc acc tgc cct cgt tcc gac ggc cgc 960
Asp Gly Thr Thr Asp Ile Thr Phe Thr Cys Pro Arg Ser Asp Gly Arg
305                 310                 315                 320

cgc gcg atg gag atc ttg aag aag ctt cag gtt cag ggc aac tgg acc 1008
Arg Ala Met Glu Ile Leu Lys Lys Leu Gln Val Gln Gly Asn Trp Thr

325                 330                 335

aat gtg ctt tac gac gac cag gtc ggc aaa gtc tcc ctc gtg ggt gct      1056
Asn Val Leu Tyr Asp Asp Gln Val Gly Lys Val Ser Leu Val Gly Ala
            340                 345                 350

ggc atg aag tct cac cca ggt gtt acc gca gag ttc atg gaa gct ctg      1104
Gly Met Lys Ser His Pro Gly Val Thr Ala Glu Phe Met Glu Ala Leu
        355                 360                 365

cgc gat gtc aac gtg aac atc gaa ttg att tcc acc tct gag att cgt      1152
Arg Asp Val Asn Val Asn Ile Glu Leu Ile Ser Thr Ser Glu Ile Arg
    370                 375                 380

att tcc gtg ctg atc cgt gaa gat gat ctg gat gct gct gca cgt gca      1200
Ile Ser Val Leu Ile Arg Glu Asp Asp Leu Asp Ala Ala Ala Arg Ala
385                 390                 395                 400

ttg cat gag cag ttc cag ctg ggc ggc gaa gac gaa gcc gtc gtt tat      1248
Leu His Glu Gln Phe Gln Leu Gly Gly Glu Asp Glu Ala Val Val Tyr
                405                 410                 415

gca ggc acc gga cgc taa                                              1266
Ala Gly Thr Gly Arg
            420

<210>  8
<211>  421
<212>  PRT
<213>  Corynebacterium glutamicum ATCC13032

<400>  8

Met Ala Leu Val Val Gln Lys Tyr Gly Gly Ser Ser Leu Glu Ser Ala
1               5                   10                  15

Glu Arg Ile Arg Asn Val Ala Glu Arg Ile Val Ala Thr Lys Lys Ala
            20                  25                  30

Gly Asn Asp Val Val Val Val Cys Ser Ala Met Gly Asp Thr Thr Asp
        35                  40                  45

Glu Leu Leu Glu Leu Ala Ala Ala Val Asn Pro Val Pro Pro Ala Arg
    50                  55                  60

Glu Met Asp Met Leu Leu Thr Ala Gly Glu Arg Ile Ser Asn Ala Leu
65                  70                  75                  80

Val Ala Met Ala Ile Glu Ser Leu Gly Ala Glu Ala Gln Ser Phe Thr
                85                  90                  95

Gly Ser Gln Ala Gly Val Leu Thr Thr Glu Arg His Gly Asn Ala Arg
            100                 105                 110

Ile Val Asp Val Thr Pro Gly Arg Val Arg Glu Ala Leu Asp Glu Gly

115                 120                 125

Lys Ile Cys Ile Val Ala Gly Phe Gln Gly Val Asn Lys Glu Thr Arg
    130                 135                 140

Asp Val Thr Thr Leu Gly Arg Gly Gly Ser Asp Thr Thr Ala Val Ala
145                 150                 155                 160

Leu Ala Ala Ala Leu Asn Ala Asp Val Cys Glu Ile Tyr Ser Asp Val
                165                 170                 175

Asp Gly Val Tyr Thr Ala Asp Pro Arg Ile Val Pro Asn Ala Gln Lys
            180                 185                 190

Leu Glu Lys Leu Ser Phe Glu Glu Met Leu Glu Leu Ala Ala Val Gly
        195                 200                 205

Ser Lys Ile Leu Val Leu Arg Ser Val Glu Tyr Ala Arg Ala Phe Asn
    210                 215                 220

Val Pro Leu Arg Val Arg Ser Ser Tyr Ser Asn Asp Pro Gly Thr Leu
225                 230                 235                 240

Ile Ala Gly Ser Met Glu Asp Ile Pro Val Glu Glu Ala Val Leu Thr
                245                 250                 255

Gly Val Ala Thr Asp Lys Ser Glu Ala Lys Val Thr Val Leu Gly Ile
            260                 265                 270

Ser Asp Lys Pro Gly Glu Ala Ala Lys Val Phe Arg Ala Leu Ala Asp
        275                 280                 285

Ala Glu Ile Asn Ile Asp Met Val Leu Gln Asn Val Ser Ser Val Glu
    290                 295                 300

Asp Gly Thr Thr Asp Ile Thr Phe Thr Cys Pro Arg Ser Asp Gly Arg
305                 310                 315                 320

Arg Ala Met Glu Ile Leu Lys Lys Leu Gln Val Gln Gly Asn Trp Thr
                325                 330                 335

Asn Val Leu Tyr Asp Asp Gln Val Gly Lys Val Ser Leu Val Gly Ala
            340                 345                 350

Gly Met Lys Ser His Pro Gly Val Thr Ala Glu Phe Met Glu Ala Leu
        355                 360                 365

Arg Asp Val Asn Val Asn Ile Glu Leu Ile Ser Thr Ser Glu Ile Arg
    370                 375                 380

Ile Ser Val Leu Ile Arg Glu Asp Asp Leu Asp Ala Ala Ala Arg Ala
385                 390                 395                 400

Leu His Glu Gln Phe Gln Leu Gly Gly Glu Asp Glu Ala Val Val Tyr
                405                 410                 415

Ala Gly Thr Gly Arg
            420

<210>  9
<211>  1613
<212>  DNA
<213>  artificial sequence

<220>
<223>  Corynebacterium glutamicum DNA equipped with recognition sites
       for the restriction endonuclease XbaI

<220>
<221>  misc_feature
<222>  (4)..(9)
<223>  recognition site for restriction endonuclease XbaI

<220>
<221>  misc_feature
<222>  (8)..(1605)
<223>  Corynebacterium glutamicum

<220>
<221>  misc_feature
<222>  (8)..(804)
<223>  sequence upstream of the site of mutation (5'-flanking sequence)

<220>
<221>  CDS
<222>  (178)..(1131)

<220>
<221>  misc_feature
<222>  (805)..(807)
<223>  gac codon for aspartic acid

<220>
<221>  misc_feature
<222>  (805)..(805)
<223>  nucleobase guanine

<220>
<221>  misc_feature
<222>  (806)..(1605)
<223>  sequence downstream of the site of mutation (3'-flanking
       sequence)

<220>
<221>  misc_feature

<222>  (1132)..(1134)
<223>  tag stop codon

<220>
<221>  misc_feature
<222>  (1135)..(1137)
<223>  tag stop codon

<220>
<221>  misc_feature
<222>  (1605)..(1610)
<223>  recognition site for restriction endonuclease XbaI

<400>  9
ggctctagat aatttttgcc caggtggttg gtttggggca gcggttgcaa ttggatgtaa      60

ttggttgttt gtcgcagtag catgggacga aagctgttag atagcatgtt gcatccctgc     120

gttggctgat tatcgctggg ttttagggtc gatagatagg ttgggagaac acgcatt        177

atg agc gat gag aac att aac gag ttt gag cag gac gag gat ctg aac       225
Met Ser Asp Glu Asn Ile Asn Glu Phe Glu Gln Asp Glu Asp Leu Asn
1               5                   10                  15

ttc ggc gcg agc ttt agt gat gaa ttc gca gat gac gat ttc gat gca       273
Phe Gly Ala Ser Phe Ser Asp Glu Phe Ala Asp Asp Asp Phe Asp Ala
            20                  25                  30

gaa gca gac gta gaa gca gat gct gct gca gag gcc tct gcc ctg gaa       321
Glu Ala Asp Val Glu Ala Asp Ala Ala Ala Glu Ala Ser Ala Leu Glu
        35                  40                  45

gct gag cag gat ctg gaa gaa gag acc cta gat gct cca gaa gaa gcc       369
Ala Glu Gln Asp Leu Glu Glu Glu Thr Leu Asp Ala Pro Glu Glu Ala
    50                  55                  60

gca gaa gaa gct cct gct gct gca gag tcc gaa gct cca gta gaa gag       417
Ala Glu Glu Ala Pro Ala Ala Ala Glu Ser Glu Ala Pro Val Glu Glu
65                  70                  75                  80

gac gaa gag gct gac agc ctt gct cag gcg gct gct gca ctt ggt gac       465
Asp Glu Glu Ala Asp Ser Leu Ala Gln Ala Ala Ala Ala Leu Gly Asp
                85                  90                  95

acc gat gag cag gac gcg gat gca gag tac aag gct cgt ctg cgt aag       513
Thr Asp Glu Gln Asp Ala Asp Ala Glu Tyr Lys Ala Arg Leu Arg Lys
            100                 105                 110

ttc act cgt gag ctg aag aag cag cct ggt gtt tgg tac atc att cag       561
Phe Thr Arg Glu Leu Lys Lys Gln Pro Gly Val Trp Tyr Ile Ile Gln
        115                 120                 125

tgc tac tcc ggc tac gag aac aag gtg aag gcg aac ctt gac atg cgt       609
Cys Tyr Ser Gly Tyr Glu Asn Lys Val Lys Ala Asn Leu Asp Met Arg
    130                 135                 140

gct cag acc ctt gag gtt gag gat gac atc ttt gag gtt gtt gtt cct       657
Ala Gln Thr Leu Glu Val Glu Asp Asp Ile Phe Glu Val Val Val Pro
145                 150                 155                 160

atc gag cag gtc act gag atc cgt gat ggt aag cgc aag ctg gtt aag       705
Ile Glu Gln Val Thr Glu Ile Arg Asp Gly Lys Arg Lys Leu Val Lys
                165                 170                 175

cgt aag ttg ctg ccg ggc tac gtt ttg gtc cgc atg gac atg aat gac 753
Arg Lys Leu Leu Pro Gly Tyr Val Leu Val Arg Met Asp Met Asn Asp
            180                 185                 190

cgc gtg tgg tct gtt gtt cgc gat aca cct ggt gtg acc agc ttt gtg 801
Arg Val Trp Ser Val Val Arg Asp Thr Pro Gly Val Thr Ser Phe Val
        195                 200                 205

ggt gac gag ggc aat gca act cct gtg aag cac cgc gat gtt gcg aag 849
Gly Asp Glu Gly Asn Ala Thr Pro Val Lys His Arg Asp Val Ala Lys
    210                 215                 220

ttc ttg atg cct cag gag cag gct gtt gtc acc ggt gag gct gct gct 897
Phe Leu Met Pro Gln Glu Gln Ala Val Val Thr Gly Glu Ala Ala Ala
225                 230                 235                 240

gcg gct gcc gag ggt gag cag gtt gtg gct atg cct acc gat acc aag 945
Ala Ala Ala Glu Gly Glu Gln Val Val Ala Met Pro Thr Asp Thr Lys
                245                 250                 255

aag cct cag gtt gct gtg gac ttc act gtt ggt gag gct gtg acc att 993
Lys Pro Gln Val Ala Val Asp Phe Thr Val Gly Glu Ala Val Thr Ile
            260                 265                 270

ctg act ggt gct ttc gct tct gtt tct gca acg att tct tct atc gat 1041
Leu Thr Gly Ala Phe Ala Ser Val Ser Ala Thr Ile Ser Ser Ile Asp
        275                 280                 285

cct gag ctg cag aag ctg gaa gtt ttg gtg tcc atc ttt ggt cgt gaa 1089
Pro Glu Leu Gln Lys Leu Glu Val Leu Val Ser Ile Phe Gly Arg Glu
    290                 295                 300

act cct gtt gat ctc agc ttc gac cag gtt gag aag gtt agc 1131
Thr Pro Val Asp Leu Ser Phe Asp Gln Val Glu Lys Val Ser
305                 310                 315

tagtagctaa actgcaccac ttaccccgca tttcctaggc cacatataag ggctttggtg 1191

atgcggggtt ttgcgtgtag ggtagacaat cgcgtgtttt ttaagcatgc tcaaaatcat 1251

tcatccccgg tggcccggtt acgtaaagat cagcaaagat gatcaactaa agcgatcatc 1311

tgaagttgta gcgggaccga gcatccggac ggttactagt ggggtttcat cgtcccagtt 1371

gtggccggta acaaggaagc aggtttaacg atggctccta agaagaagaa gaaggtcact 1431

ggcctcatca agctccagat ccaggcagga caggcaaacc ctgctcctcc agttggccca 1491

gcacttggtg ctcacggcgt caacatcatg gaattctgca aggcttacaa cgctgcgact 1551

gaaaaccagc gcggcaacgt tgttcctgtt gagatcaccg tttacgaaga ccgtctagag 1611

cc 1613

<210> 10
<211> 318
<212> PRT
<213> artificial sequence

<220>
<223> Synthetic Construct

<400>  10
   Met Ser Asp Glu Asn Ile Asn Glu Phe Glu Gln Asp Glu Asp Leu Asn
   1               5                   10                  15
   Phe Gly Ala Ser Phe Ser Asp Glu Phe Ala Asp Asp Asp Phe Asp Ala
               20                  25                  30
   Glu Ala Asp Val Glu Ala Asp Ala Ala Ala Glu Ala Ser Ala Leu Glu
           35                  40                  45
   Ala Glu Gln Asp Leu Glu Glu Glu Thr Leu Asp Ala Pro Glu Glu Ala
       50                  55                  60
   Ala Glu Glu Ala Pro Ala Ala Ala Glu Ser Glu Ala Pro Val Glu Glu
   65                  70                  75                  80
   Asp Glu Glu Ala Asp Ser Leu Ala Gln Ala Ala Ala Ala Leu Gly Asp
                   85                  90                  95
   Thr Asp Glu Gln Asp Ala Asp Ala Glu Tyr Lys Ala Arg Leu Arg Lys
               100                 105                 110
   Phe Thr Arg Glu Leu Lys Lys Gln Pro Gly Val Trp Tyr Ile Ile Gln
           115                 120                 125
   Cys Tyr Ser Gly Tyr Glu Asn Lys Val Lys Ala Asn Leu Asp Met Arg
       130                 135                 140
   Ala Gln Thr Leu Glu Val Glu Asp Asp Ile Phe Glu Val Val Val Pro
   145                 150                 155                 160
   Ile Glu Gln Val Thr Glu Ile Arg Asp Gly Lys Arg Lys Leu Val Lys
                   165                 170                 175
   Arg Lys Leu Leu Pro Gly Tyr Val Leu Val Arg Met Asp Met Asn Asp
               180                 185                 190
   Arg Val Trp Ser Val Val Arg Asp Thr Pro Gly Val Thr Ser Phe Val
           195                 200                 205
   Gly Asp Glu Gly Asn Ala Thr Pro Val Lys His Arg Asp Val Ala Lys
       210                 215                 220
   Phe Leu Met Pro Gln Glu Gln Ala Val Val Thr Gly Glu Ala Ala Ala
   225                 230                 235                 240
   Ala Ala Ala Glu Gly Glu Gln Val Val Ala Met Pro Thr Asp Thr Lys
   245 250 255
   Lys Pro Gln Val Ala Val Asp Phe Thr Val Gly Glu Ala Val Thr Ile
   260 265 270
   Leu Thr Gly Ala Phe Ala Ser Val Ser Ala Thr Ile Ser Ser Ile Asp
   275 280 285
   Pro Glu Leu Gln Lys Leu Glu Val Leu Val Ser Ile Phe Gly Arg Glu
   290 295 300
   Thr Pro Val Asp Leu Ser Phe Asp Gln Val Glu Lys Val Ser
   305 310 315

<210>  11
<211>  20
<212>  DNA
<213>  artificial sequence

<220>
<223>  primer N210D_for

<400>  11
accttgacat gcgtgctcag                                                  20

<210>  12
<211>  20
<212>  DNA
<213>  artificial sequence

<220>
<223>  primer N210D_rev

<400>  12
catagccaca acctgctcac                                                  20

<210>  13
<211>  20
<212>  DNA
<213>  artificial sequence

<220>
<223>  probe NCgl0458_N210D_C

<400>  13
tgccctcgtc acccacaaag                                                  20

<210>  14
<211>  35
<212>  DNA
<213>  artificial sequence

<220>
<223>  probe NCgl0458_N210D_A

<400>  14
aacttcgcaa catcgcggtg cttcacagga gttgc                                35

201800170A       21